# EUROPEAN PATENT SPECIFICATION

(11) **EP 1 827 087 B1**
(45) Date of publication and mention of the grant of the patent: **08.08.2012**
(21) Application number: 05818667.7
(22) Date of filing: 13.12.2005
(51) Int. Cl.: A01K 67/027, C12N 15/09, G01N 33/50

(54) **TRANSGENIC ANIMALS FOR ASSESSING DRUG METABOLISM AND TOXICITY**
VERWENDUNG TRANSGENER TIERE UM DEN METABOLISMUS UND DIE TOXIZITÄT VON MEDIKAMENTEN ZU ANALYSIEREN
ANIMAUX TRANSGENIQUES POUR EVALUER LE METABOLISME ET LA TOXICITE D'UN MEDICAMENT

(30) Priority: 13.12.2004 GB 0427172; 05.08.2005 GB 0516187; 10.08.2005 US 707077 P
(43) Date of publication of application: 05.09.2007
(73) Proprietor: ITI Scotland Limited, Glasgow G2 5SG (GB)
(72) Inventor: WOLF, Charles R., CXR Biosciences Ltd., Dundee, DD1 5JJ (GB); SCHEER, Nico, Artemis Pharmaceuticals GmbH, D-51063 Koeln (DE); FAUST, Nicole, Artemis Pharmaceuticals GmbH, D-51063 Koeln (DE)
(74) Representative: Goodfellow, Hugh Robin
(86) International application number: PCT/GB2005/004772
(87) International publication number: WO 2006/064197

(56) References cited:
- WO-A-02/083897
- XIE WEN ET AL: "Humanized xenobiotic response in mice expressing nuclear receptor SXR" NATURE, NATURE PUBLISHING GROUP, LONDON, GB, vol. 406, no. 6794, 27 July 2000 (2000-07-27), pages 435-439, XP002166094 ISSN: 0028-0836 cited in the application
- ROBERTSON G R ET AL: "Transgenic Mouse Models of Human CYP3A4 Gene Regulation" MOLECULAR PHARMACOLOGY, BALTIMORE, MD, US, vol. 64, no. 1, 2003, pages 42-50, XP002981929 ISSN: 0026-895X
- ZHANG J ET AL: "Modulation of Acetaminophen-Induced Hepatotoxicity by the Xenobiotic Receptor CAR" SCIENCE, AMERICAN ASSOCIATION FOR THE ADVANCEMENT OF SCIENCE,, US, vol. 298, 11 October 2002 (2002-10-11), pages 422-424, XP002981927 ISSN: 0036-8075 cited in the application
- ZHANG WEISHENG ET AL: "In vivo activation of the human CYP3A4 promoter in mouse liver and regulation by pregnane X receptors." BIOCHEMICAL PHARMACOLOGY, vol. 65, no. 11, 1 June 2003 (2003-06-01), pages 1889-1896, XP002370456 ISSN: 0006-2952

## Description

The present invention relates to transgenic mice tissues or cells derived therefrom and methods of producing them. The transgenic mice or tissues or cells derived therefrom provide a system capable of expressing human proteins responsible for drug metabolism in place of the homologous endogenous mouse proteins and for the controlled expression of human genes introduced into the animal so that the expression of the human genes is regulated in a manner more closely analogous to that seen *in vivo* in humans. The transgenic mice or tissues or cells derived therefrom are for use, especially but not exclusively, in assessing xenobiotic or drug metabolism, toxicity or other properties or functions of the introduced human proteins such as metabolism and/or biosynthesis of endogenous compounds.

### Background to the Invention

A significant proportion of therapeutic drug candidates fail to become marketable drugs because of adverse metabolism or toxicity discovered during clinical trials. These failures represent a very significant waste of development expenditure and consequently there is a need for new technologies that can more reliably, quickly and economically predict at the pre-clinical development stage the metabolic and toxicological characteristics of drug candidates in man. At present, most are-clinical metabolic and toxicity testing of drug candidates relies on laboratory animals, human and/or mammalian cell lines and/or tissues in culture. However, none of these methods is completely reliable in predicting metabolism or toxicity in a human subject. Metabolic and toxicological data from animals can differ significantly from that obtained front a human subject due to species differences in the biochemical mechanisms involved. In addition, interpretation of data derived from *in vitro* human cell cultures or isolated human tissue studies can be problematic since such systems are not available for all organs and tissues or they fail to retain the same metabolic characteristics as they possess *in vivo*.

It is known in the prior art that the metabolism, distribution and toxicity of most drugs depends on their interactions with four distinct main classes of proteins:
a) Phase-1 drug-metabolising enzymes, such as the cytochromes P450 which generally add or expose polar groups on the xenobiotic molecule;
b) Phase-2 drug-metabolising enzymes, such as transferases, in particular the glucuronyl transferases, glutathione transferases, sulphonyl transferases and acetyl transferases which conjugate the polarised xenobiotic molecule to a hydrophilic group thereby facilitating its subsequent excretion;
c) Drug transporter proteins, such as the ATP-binding cassette proteins which include the multi-drug resistance proteins (MDRs) and multi-drug resistance-associated proteins (MRPs) and the organic anion transporting polypeptides (OATPs) which facilitate the transport of drugs and other xenobiotic molecules across plasma membranes;
d) Transcription factors, such as the pregnane X receptor (PXR) and the constitutive androstane receptor (CAR) which regulate the transcription of genes encoding proteins of the preceding classes, in particular the cytochromes P450.

Variation between species is known in each of these protein classes both with respect to the multiplicity of proteins within each class, the function of the proteins themselves and with respect to genetic regulation of their expression.

It is known from WO2004/007708 how to produce non-human transgenic animals expressing functioning human P450s in which the functions of endogenous cytochromes P450 have been annulled by deletion of individual P450 genes or by deletion of the cytochrome P450 reductase gene encoding the enzyme on which the function of all cytochromes P450 depends. However, the described animal model has limitations in that not only are the introduced P450s restricted to particular organs or tissues of the non-human animal but also there is no provision to regulate expression of the human P450s in a manner that is analogous to that seen in the human. One of the prior art models is also limited in that additional modifications are needed to provide cytochrome P450 reductase activity to the introduced human P450s without reactivating endogenous non-human P450s. A yet further disadvantage resides in the lack of provision to reproduce human phase-2 metabolism, thus the system is unable to provide an entire metabolic profile.

It is also known from the prior art to humanise the induction characteristics of cytochromes P450 in the mouse by expressing human PXR (Xie et al, Nature Vol 406, 435-9, 2000) or human CAR (Zhang et al, Science Vol 298, 422-4, 2002) in a mouse wherein the mouse PXR gene and/or mouse CAR gene respectively have been deleted. While such animals demonstrate induction patterns of endogenous P450s that reproduce those seen in the human they have undesirable characteristics because the cytochromes P450 whose expression is regulated analogously to the human are still non-human cytochromes P450. A further disadvantage is that because the PXR or CAR genes themselves are not regulated as they are in the human by virtue of the transgene being driven by a heterologous tissue-specific promoter (albumin promoter), over-expression of the heterologous gene can occur which can have the result that a normal metabolic pathway is bypassed. Moreover, the PXR and CAR transgenes are derived from a cDNA rather than a genomic clone, thus the transgenic non-human animals consequently lack the sequences necessary correctly to reproduce all the transcriptional and post-transcriptional regulation of PXR or CAR expression hence their expression is restricted to the liver and may not be of a physiological level. In addition these models do not encode for splice variants of the human gene. Another drawback of the PXR/CAR models is that they are unsuitable to combine with modifications of other genes within one animal since the humanisation of each gene is achieved by two independent genomic alterations: (i) knock-out of the endogenous gene (ii) transgenesis with the human orthologue under control of the albumin promoter at a different genomic location.

There is therefore a need for improvements in animal models of human metabolism that can control expression of the human genes introduced into the animal so that their expression is regulated in a manner more closely analogous to that seen in humans. There is also a need for more aspects of the human metabolic pathway to be reproduced. Effective animal models of human metabolism require not only expression of the relevant human proteins but also annulment of the functions of the homologous endogenous proteins.

One reason why the present invention embodies a surprising advance over the prior art is that many prior art researchers appear to be of the view that the problem posed by the need for models of drug metabolism is already solved. For example, Xie and Evans (2002, DDT7, p509) state that humanizing PXR is "one of the rare examples where replacing a single transcriptional regulator allows conversion of species-specific gene regulation". Furthermore, it is evident that workers have turned their attention to techniques that differ markedly to those that utilise transgenic animal systems. For example, attempts are being made to humanise the mouse liver as an organ by using human hepatocytes, the aim being to obtain a mouse model for drug metabolism in humans. This work is labour-intensive though, and in the inventors' opinion is of dubious relevance to the situation in reality.

The present invention is the first methodology that takes into account all of the problems that prior art systems suffer from and that seeks to resolve these problems in a practical manner. The inventors have recognised that in order to provide transgenic mouse models with humanised drug metabolism pathways that overcome the undesirable features of the animal models described in the prior art a number of criteria should ideally be satisfied:
a) Regulation of the expression of introduced human proteins such that patterns of expression in the human are reproduced;
b) Expression of multiple human proteins so that multiple aspects of human metabolism are reproduced;
c) Annulled expression or function of multiple endogenous genes so that interference. from non-human metabolic pathways on the functions of introduced human proteins is significantly reduced.

In the present invention, we provide methods of producing mouse cell and transgenic mice that incorporate at least some if not all of these desired qualities. Such mouse cell and transgenic mice possess desirable characteristics not available in the prior art in that they can model entire human pathways of xenobiotic metabolism rather than just individual elements of pathways and that such models are provided for all tissues and organs. This is achieved through the application of technical approaches hitherto not available in the prior art with respect to obtaining regulation of transgene expression analogous to that seen in human cells through the use of extensive regulatory DNA sequences and with respect to annulment of endogenous metabolic pathways through deletion or gene exchange. A number of relevant human proteins are expressed in a single animal.

### Statement of the Invention

According to a first aspect of the invention there is provided a transgenic mouse, tissue or cells derived therefrom incorporating in its genome at least one human DNA sequence encoding at least one transcription factor under the control of a transcription factor promoter and whose endogenous equivalent genes have been annulled, the mouse, tissue or cells derived therefrom further incorporating at least one or more of the following further human DNA sequences selected from the group comprising:
(i) a DNA sequence encoding a phase-1 drug-metabolising enzyme;
(ii) a DNA sequence encoding a phase-2 drug-metabolising enzyme; and/or
(iii) a DNA sequence encoding a drug transporter protein
and whose endogenous equivalent gene or genes have been annulled;
wherein the human sequence encoding the at least one transcription factor is inserted at the point in the genome where the endogenous equivalent gene naturally occurs; and wherein the transcription factor is selected from the group comprising the pregnane X receptor (PXR/SXR) and the constitutive androstane receptor (CAR) or a combination thereof.

Throughout the specification and the claims which follow, unless the context requires otherwise, the word "comprise", or variations such as "comprises" or "comprising" will be understood to imply the inclusion of a stated integer or group of integers but not the exclusion of any other integer or group of integers.

Reference herein to "endogenous equivalent gene" of the non-human animal is intended to include a gene or genes whose expression product retains the same, similar or identical function as the human counterpart gene. For example, the human transcription factor gene known as PXR (NR1I2 nuclear receptor subfamily 1, group I, member 2), Entrez GeneID: 8856, has a murine counterpart of the same name whose Entrez GeneID is 18171. The proteins encoded by these genes have an equivalent function in the organisms from which they are derived.

Generally, the introduced transcription factor gene, the phase-1 drug-metabolising enzyme gene, the phase-2 drug-metabolising enzyme gene and/or the drug transporter protein gene will share a degree of homology with the endogenous gene with which it is equivalent. Preferably, the degree of homology will be greater than 30%, greater than 40%, greater than 50%, greater than 60%, greater than 70%, greater than 80%, greater than 90%, or even greater than 95%.

In the case of drug-metabolising enzyme genes, equivalence between genes can be assessed by a combination of substrate specificity, mode of regulation (for example, by transcription factors or exogenous drugs), sequence homology and tissue distribution. Certain genes have exact equivalents; examples of such genes are CYP2E1, CYP1A1, CYPIA2. CYP2B6 and CYP2D are examples where there is only one gene in the human, but numerous equivalent genes in the mouse. There are four CYP2C genes in the human, and numerous equivalent genes in the mouse. In such circumstances, at least one, preferably two, three, four, five or more or even all of the equivalent murine genes are annulled. CYP3A4 is an example where there is no obvious orthologue in the mouse, but CYP3A11 could be considered at least one equivalent mouse gene because of its hepatic expression, mode of regulation and sequence homology.

Reference herein to "annulled" is intended to include silencing or deletion or rendering inactive so that the non-human animal's endogenous equivalent gene is unable to express the gene product(s), at least not to any level that is significant to the drug metabolism process. For instance, the expression level of an annulled gene may be less than 20%, preferably less than 10%, more preferably less than 5%, more preferably less than 2%, even more preferably 1% or less of the wild type expression level. The expression of an annulled gene may preferably be decreased to the point at which it cannot be detected.

### Partial genomic sequences

In the prior art techniques disclosed to date, researchers have generated transgenic models that incorporate human genes into non-human systems, notably the mouse. However, in devising these systems, little attention has been paid to retaining the context of the human gene as it exists in its natural state.

Generally, cDNA sequences have been used in the prior art rather than respecting the intron/exon structure of the human gene, or incorporating the human gene in its entirety, containing both intronic and exonic sequences. This means that any splice variants that might naturally be generated, cannot form. Splice variants are important for a number of reasons. First, they may have a function. Second, they may have a dominant negative effect, for example, by binding to their usual protein partners and altering biological effects of the protein. Third, they may sequester ligand. A system that accurately reflects the *in vivo* situation therefore preferably mirrors the balance of splice variants that exist in any biological system.

Using cDNA also means that mRNA levels are artificially generated and may not reflect the reality of the natural physiological situation.

In contrast, the present invention attempts to mirror the *in vivo* situation by providing the gene in its entirety where this is possible. This means that the intron-exon junctions are retained as in the natural system so that splicing events can happen exactly as in the natural situation. Where, perhaps because of the length of a gene, it is not simple to transpose the entire gene into a transgenic system, the invention seeks to use a combination of cDNA and genomic DNA in its constructs so that important intron-exon boundaries, where the majority of splicing events occur, are retained.

According to the invention, therefore, where it is known that the majority of splice variants occur as a result of splicing variation within a particular intron, this intron is preferably incorporated as genomic DNA in the construct, while less influential intronic sequences are not retained. This has the result that levels of functional mRNA and functional protein mirror the levels that are found *in vivo* in response to exposure to a particular drug or drug cocktail. This is what is ideally required for a physiologically-relevant model.

Accordingly, whilst cDNA sequences may be used, in preference to these sequences, the invention may use a combination of cDNA and genomic sequences from the gene that is to be humanised. For example, in the case of a transgenic animal expressing the human PXR gene, due to the large size of more than 35kb of the human PXR gene, the intron-exon structure between exons 4 and 6 is preferably maintained, since most splice variants are observed in this genomic region, since it is located within the ligand-binding domain. This advantageously retains the sequence where most splice variants are observed and is conveniently located within the ligand-binding domain.

Another example is provided by the case of the humanization cassette preferably used for CYP3A4: this may contain the 13kb human CYP3A4 promoter, exon 1 and intron 1 as in the normal genomic constitution and a human cDNA consisting of exons 2-13.

Another example is provided by the case of the humanization cassette preferably used for CYP2C9: this may contain the 12kb human CYP2C9 promoter, a human cDNA of exons 1-4, intron 4 and a cDNA of exons 5-9.

### Complete genomic sequences

Complete genomic DNA sequences may be used. For instance, in the case of a transgenic animal expressing the human CAR gene, the relatively small size of the human CAR, which comprises roughly 7kb from exon 2-9, makes it simple to retain the complete genomic structure in the targeting vector. The construct should preferably retain the intron-exon structure between exons 2 and 9. This advantageously retains the complete genomic structure within the targeting vector and permits coverage of all splice variants of human CAR. Preferably, the genomic human CAR sequence is fused to the translational start site of the mouse CAR gene. The human CAR sequence then contains all genomic sequences of exons 1-9. The 5' and 3'UTRs may be human or may be retained from the mouse genome. All other parts of the coding sequences of the mouse CAR gene can be deleted.

### Transcription factors

The human DNA encoding a transcription factor is selected from the group comprising the pregnane X receptor (PXR, also known as the steroid and xenobiotic receptor SXR) and the constitutive androstane receptor (CAR) or multiples thereof or a combination thereof. Mice and cells according to this aspect of the invention are advantageous for the reasons described in detail above. For example, recent evidence further supports the contention that the ligand binding domains of the murine and human CAR proteins are divergent relative to other nuclear hormone receptors, resulting in species-specific differences in xenobiotic responses (Huang et al., 2004, Molecular endocrinology 18(10):2402-2408). Results reported in this paper demonstrate that a single compound can induce opposite xenobiotic responses via orthologous receptors in rodents and humans.

Transgenic mice for human PXR have been created and are described in the examples included herein. Human PXR is found to be expressed in both the liver and GI tract of mice in the predicted manner at levels equivalent to those of the endogenous mouse gene. In this way, typical problems faced by conventional techniques of this type, such as over- or under-expression are avoided. Furthermore, currently available humanised PXR models use the albumin promoter to drive human PXR which has been crossed into a PXR-null background. Therefore, PXR is expressed at very high levels in this model and there is no PXR in any tissue other than the liver. This severely compromises the use of this model to understand the role of hPXR in controlling gene expression in the GI tract or at the blood brain barrier or, indeed, in any other tissue.

In this model, the PXR protein has also been shown to be functional as the mice are responsive to compounds such as rifampicin and TCPOBOP that are known to induce gene expression via this pathway. Strain differences between wild type and the humanised mice have been demonstrated. For example, the humanised mice are shown to be more responsive to compounds such as rifampicin, that are known to be more active to hPXR. Humanised PXR animals thus demonstrated an altered sensitivity to rifampicin relative to the wild type.

Furthermore, there was clearly greater background P450 enzyme activity as measured by 16-beta-hydroxylation of testosterone and 7-benzyloxyquinoline demethylation between wild type and humanised PXR mice.

In experiments using the inducing agent TCPOBOP, the hepatic microsomal metabolism of testosterone was measured. Again clear differences between the wild type and the humanised PXR animals were observed. In particular, the 7-alpha-hydroxylation of testosterone was constitutively higher in the huPXR animals relative to the wild types.

Consistent with the strain differences in wild type and human PXR, there were marked differences in the sensitivity of the mouse lines to induction by TCPOBOP. In the case of testosterone 16-alpha-hydroxylation, this activity was significantly induced in wild type animals but not in humanised PXR animals. Of particular interest was the observation that the induction of testosterone 16-beta-hydroxylation was much more marked in wild type than in huPXR animals. Indeed, at a dose of Img/kg, induction of testosterone 16-beta-hydroxylation was approximately 6-fold in wild type animals but only 1.7-ford in the huPXR animals. This again demonstrates a reduced sensitivity of the humanized mice relative to controls.

Transgenic mice and cells according to this aspect of the invention preferably demonstrate the functional properties described above. For example, such cells and mice preferably do not display induction of Cyp2b10 activity in response to rifampicin. However, such cells and mice do display an induction effect for CYP3A11, not only with rifampicin but also for TCPOBOP.

It will be appreciated that other human DNA(s) encoding a transcription factor may also be used in the present invention providing that they are capable of regulating a phase-1 drug-metabolising enzyme, a phase-2 drog-metabolising enzyme and/or a drug transporter protein. Examples include PPARs (a, δ and γ), NRF2, the Ah receptor, HNF1 and HNF4.

Preferably, the human DNA encoding a transcription factor comprises both the pregnane X receptor (PXR) and the constitutive androstane receptor (CAR). In this embodiment of the invention the transgenic mouse or tissue or cells derived therefrom may be considered as "double-humanised" for these transcription factor genes. Such double-humanised models are advantageous over models that only incorporate a single gene (either PXR or CAR) because many drug metabolising enzymes or drug transporters possess elements that are responsive to the binding of both CAR and PXR. Furthermore, the numbers of PXR-responsive elements often differ from the numbers of CAR-responsive elements and so regulation by both transcription factors is generally important. Consequently, models that take account of the effects of both factors are preferable and more closely mirror the physiological situation in vivo.

Mice transgenic for both human PXR and human CAR have been created and are described in the examples included herein. Preliminary studies have been performed on the activity of these transcription factors in combination, determined by measuring barbiturate-induced sleeping time. Sleeping time has been known for many years to be directly proportional to the hepatic cytochrome P450 activity and this activity can be at least in part ascribed to the P450 levels in the liver determined by CAR and PXR function. Whereas wild type mice given a narcotic dose of pentobarbitone slept for 21 minutes, the double humanized mice for CAR and PXR slept for 34 minutes. These mice therefore demonstrate a significant difference to their wild type controls indicating that the double humanised mouse has a marked difference in its response to drugs relative to the wild type animals.

Transgenic mice and cells according to this aspect of the invention preferably demonstrate the functional properties described above. For example, cells and mice transgenic for human PXR preferably do not display induction of Cyp2b10 activity in response to rifampicin, but do display an induction effect for CYP3A11, not only with rifampicin but also for TCPOBOP.

The inventors have also noted that the capacity of promoters to induce enzyme expression is different in different tissues. Accordingly, it is of utmost importance for human transcription factors to be used rather than endogenous transcription factors from another animal. Furthermore, the regulatory sequences of the transcription factors and the genes that they regulate should mirror the natural physiological situation as closely as possible. The use of as many human transcription factors, human drug metabolising enzymes and human drug transporters as possible is important to ensure that this happens.

The ratio of protein levels that are generated by a particular drug are also of significant importance. For example, the action of mouse PXR stimulates expression of different proteins than the action of human PXR and at different levels. The levels of a particular drug and its metabolites depends crucially on which drug metabolising enzymes and transporters are expressed and so, again, it is of utmost importance for human transcription factors to be used rather than endogenous transcription factors from another animal.

The use of human transcription factors is also important from a toxicological standpoint. For example, PXR is naturally regulated by bile acids and other physiological compounds and toxic conditions such as biliary necrosis and biliary cholestasis can result from exposure to a particular drug. It may therefore be that as a result of differences between drug metabolism between human and a test animal, a toxic effect will be noted in that animal that would not be evident in the human.

The regulatory sequences governing expression of the transcription factor(s) may preferably be either of human origin, or may originate from the target animal species, the mouse.

### Phase-1 drug-metabolising enzymes

Preferably, the human DNA encoding a phase-1 drug-metabolising enzyme is selected from the group comprising the cytochromes P450, including but not limited to CYP3A4, CYP2B6, CYP2C9, CYP2C19, CYP2D6, and CYP2B6. It will be appreciated that other DNAs encoding a phase-1 drug-metabolising enzyme may also be included in the present invention, providing that they are capable of modifying a xenobiotic by adding or exposing a polar group on the xenobiotic molecule.

The choice of human P450 isoforms for introduction into P450-humanised mice is predominantly driven by the known relative importance of various P450 isoforms in metabolism in the relevant tissue. Thus, for example, to date the single most significant P450 isoform recognised in the human liver is CYP3A4, and so CYP3A4 is therefore probably the first human P450 isoform of choice for P450 humanisation of liver. The choice of human P450s for the multi-P450-humanised mouse of the present invention is dictated by the need of the user. In this respect it is expected that any one or more of the following human isoforms will be preferred: 3A4, 2D6, 2B6, 2C9, 2C19, 1A2, 2C8. However, it will be appreciated that the isoform(s) incorporated into the animal cell is/are dependent on the user's requirement. In this way, the humanised transgenic animal may be "designed" to investigate the role of specific isoforms in the metabolic process.

According to the teaching of the invention, single genes, gene clusters or combinations of single genes or gene clusters may be replaced. Whole gene clusters should preferably be replaced where possible, rather than simply replacing individual genes. This generates a situation in which the ratios of the expression levels of genes in any gene cluster are the same as the ratios in which these genes are expressed is vivo. This is a phenomenon that has not received attention in the prior art. The CYP3A and CYP2C clusters are preferred clusters of phase-1 drug-metabolising enzymes for replacement according to the present invention. Either one, or even both of these gene clusters may be replaced according to the present invention.

According to the invention, therefore, partial, or preferably complete cascades of genes that are implicated in a particular pathway may preferably be replaced. This ensures that the partial redundancy of gene function is retained and so, again, the real physiological situation is mirrored. An example can be provided by the CYP3A P450 cluster. In humans, there are four functional genes in this cluster, that have overlapping substrate specificity. The CYP3A5 protein will, for example, metabolise CYP3A4 substrates. Therefore, if one is attempting to generate a model of drug metabolism, incorporating the CYP3A5 gene in any state other than as part of its entire gene cluster is distancing the generated model from reality.

In contrast, following prior art teachings, were a researcher to decide that it might be a good idea to generate an animal transgenic for the CYP3A5 gene, he would only know to incorporate this gene, and would not think to incorporate the entire gene cluster of which it forms a part. Furthermore, using prior art techniques, it would insert at an arbitrary site and not in its natural contextual position in the genome. Worse still, its expression would be under the direction of a strong promoter specific for the liver. This strategy would very likely generate a large amount of CYP3A5 protein in the liver, and because of the large amounts of protein provided, this protein would metabolise substrates at a greater rate than those on which it would act *in vivo*. For all these reasons, the method of the invention is advantageous over systems that have been described previously.

Preferably, the clusters of phase 1 drug metabolising enzymes that are used for humanisation are the CYP3A cluster and the CYP2C cluster.

In view of the redundancy in protein function between the human and commonly used target transgenic animals such as the mouse, humanisation for phase 1 drug metabolising enzymes is preferably performed against a deleted background in which only some (for example 1, 2, 3, 4 or 5), and preferably none of the target animal phase 1 drug metabolising enzymes are expressed at significant levels.

### Phase-2 drug-metabolising enzymes

Preferably, the human DNA encoding a phase-2 drug-metabolising enzyme is selected from the group comprising the glucuronyl transferases, for instance, the UGT1A gene or gene cluster, the glutathione transferases, for instance GST (glutathione S-transferases), the sulphonyl transferases and the acetyl transferases. It will be appreciated that other DNAs encoding a phase-2 drug-metabolising enzyme may also be included in the present invention providing that they are capable of conjugating a product of phase-1 metabolism.

Preferably, a cluster of phase 2 drug metabolising enzymes that is used for humanisation is the UGT1A gene cluster.

### Drug transporters

Preferably, the human DNA encoding a drug transporter protein is selected from the group comprising the ATP-binding cassette proteins which include but are not limited to the multi-drug resistance proteins, for instance MDR-1 and multi-drug resistance-associated proteins (MRPs), for example, MRP1 and/or MRP2, or from the organic anion transporting polypeptides (OATPs). It will be appreciated that other DNAs encoding a drug transporter protein may also be included in the present invention providing that they are capable of facilitating the transport of drugs and other xenobiotic molecules across plasma membranes.

Preferably, the multidrug resistance protein is MDR1.

Preferably, the multi-drug resistance-associated protein is MRP2.

### Regulatory sequences

The present invention resides in the humanisation of transgenic mouse cell, tissue or animals especially for transcriptional factor(s), wherein the transcriptional factor transgenes are driven by transcriptional factor promoters, that is to say they are "knocked-in" rather than utilising heterologous albumin/tissue specific promoters. Thus the mice of the present invention are able to express the human proteins at not only the appropriate physiological levels but in all tissues, rather than just the liver as is known from the prior art.

It will be appreciated that any human DNA sequences include coding sequences for proteins selected from the group of classes of human phase-1 metabolism enzymes; human phase-2 metabolism enzymes; human drug transporters; human transcription factors, may ideally be operatively linked to human regulatory DNA sequences.

Preferably, these human DNA sequences of the above-described transcription factors and other proteins are whole genes or are DNA constructs comprising regulatory genes that may either be derived from humans or animals. In cases where the regulatory genes are not of human origin, the regulatory genes may be derived from the target animal, for example, the mouse. By regulatory genes is meant to include any promoter or enhancer sequences, 5' or 3' UTRs, poly-A termination sequences or other DNA sequences, that are necessary for transcription of the gene of interest Transcripts used for insertion of human sequences are preferably terminated by a poly A motif.

Heterologous promoters have generally been used in the prior art, and those used (such as the albumin promoter) are generally strong promoters, are ligand independent in their action and are constitutively switched on. In normal development, albumin is only expressed neo-natally. This divorces the expression of the protein encoded by the gene from the natural situation in reality, in that the regulatory signals that direct transcription of the gene and the subsequent translation of the mRNA product are not retained in the transgenic system. Researchers in the prior art turned to the use of such promoters for a variety of reasons. Partly, it was felt necessary to do so because the transcription signals provided by the endogenous promoters were not deemed to be strong enough. Furthermore, it was thought necessary to use promoters that had been shown to be effective in the mouse.

In contrast, the invention preferably incorporates the endogenous promoter with the human gene so that the fidelity of wild type human expression is retained, developmentally, temporally and in a tissue-specific manner.

By "endogenous promoter" is meant the promoter that naturally directs expression of the gene of interest. The endogenous promoter may thus be the endogenous human promoter, or may alternatively be the promoter that is endogenous to that introduced gene in the transgenic mouse subject. In transgenic mice, the expression of the human gene may be directed by the endogenous mouse promoter for that gene. By incorporation of all of the 5' upstream sequence that is necessary for promoter activity, preferably including any enhancers, it has been found that it is not in fact necessary to use strong, constitutive promoters; as in the natural situation, the endogenous promoter, in its entirety, is perfectly capable of directing expression of the relevant protein in a physiologically relevant manner. An example is provided by the CYP3A4 gene, which possesses strong enhancer elements up to 13kb upstream of the transcription initiation point. Whilst incorporation of all this sequence allows the appropriate mechanism of transcription to occur, omission of these upstream sequences leads to a system in which incomplete or insufficient regulatory sequences are present to allow the fidelity of gene expression to be retained.

One result of using promoters such as the albumin promoter that were exclusively, used in the prior art is that the effects of expression of a gene can only be monitored in a particular tissue - in the case of the albumin promoter, this is the liver. Prior art workers were not discouraged by this limitation, because the liver was generally viewed as being the only important tissue for studying drug metabolism, and therefore only expression in the liver was desired. Expression elsewhere in tissues other than the liver was seen as artefactual and therefore a hindrance to an effective model rather than being in any way advantageous. Another drawback of the prior art systems based on the use of the albumen promoter is that for that system to work, a murine PXR null background is required. This means that PXR is not expressed anywhere other than from the transgene in the liver, which has very wide-ranging effects on drug metabolism; such a mouse no longer reflects the natural tissue distribution of a natural mouse.

The inventors are of the view that the liver is not the only important tissue for drug metabolism. Accordingly, what the prior art workers perceived as an advantage, i.e. that exclusive liver-specific expression enabled an accurate assessment of the real physiological situation, the inventors see as a distinct disadvantage because other potentially important tissues are ignored. The invention allows a global, holistic snapshot to be obtained of the drug metabolism process.

Use of the endogenous promoter also carries other advantages with it. In particular, the fidelity of developmental expression is retained. Whereas prior art systems have used liver-specific promoters that sponsor liver expression exclusively, the use of the natural endogenous promoter ensures that the protein is expressed in the tissues in which it naturally occurs, and not only in the adult animal, but also at each developmental stage. This also carries with it the advantage that the transgenic animals are more likely to be viable and thus useful as drug screens and in the development of downstream crosses. It also allows the animals to be used to screen for teratogenic effects of a test compound, as placental expression of transcription factors and drug metabolising enzymes is retained.

Furthermore, the inventors have also noted the existence of a potential "repression" effect whereby a particular drug compound reduces the level of a particular drug transporter or metabolising enzyme and so alters the rate or pathway of disposition. For example, were the levels of human CYP3A4 to decrease, for example as a result of repression of mouse PXR rather than the conventional human transcription factor partner, then an alternative pathway of disposition may be exaggerated. This would give a misleading impression of the enzyme levels that are induced by a particular drug in an organism. It would also give a misleading impression of the rate and type of metabolism that would operate in the human on exposure to that particular drug. The inventors have also noted that the duration of induced expression by a particular drug is of great importance. For example, some drugs that are candidates for use in humans may not be metabolised efficiently in the mouse. This means that such a drug remains present at a systemically high concentration for a significant period. This means that transcription factors such as PXR will remain activated for this period, being constantly activated for this period. Associated levels of drug metabolising enzymes, drug transporters and other such enzymes will as a result also be highly expressed during the entire period that the drug remains in the animal. This clearly is misleading and in contrast to the equivalent situation in the human where the metabolism of the drug may be significantly more efficient.

Using the human promoter rather than the mouse promoter may be preferable to drive the expression of transcription factors, phase 1 drug metabolising enzymes, phase 2 drug metabolising enzymes, and/or drug transporters, as it allows the idiosyncrasies of the human expression system to be retained. Again using the example of the mouse CYP3A genes, these have a different number of PXR and CAR response elements to the number that is present in the human CYP3A4 gene. Were the equivalent mouse promoter to be used, then the response to transcriptional activation of PXR by exposure of the animal to drug would be correspondingly different to the response that is evident in a human system. It is also true that in many cases, the benefit of using the human promoter is that a true mouse orthologue does not exist. This would then lead to greater or lesser production of the drug metabolising protein in response to a particular drug, so potentially exaggerating or diminishing the role of that protein in the metabolism of that drug. For example, in a particular mouse system known in the prior art, it may be that because of inappropriately high levels of expression of CYP3A4, driven by inappropriate an promoter, the tested drug is metabolised too quickly for any potentially beneficial effect to be evident. This is of course misleading.

In contrast, using the natural human promoter means that, following this example, the appropriate amount of CYP3A4 would be produced in response to drug activation, and furthermore, would be produced in the correct tissue. The natural physiological response to a particular drug will then be mimicked, in terms of the amount of CYP3A4 that is produced, not only in the liver, but also, say, in the gastro-intestinal tract. If the drug is in fact a substrate for CYP3A4, then it will be metabolised at a rate and in a manner that mirrors the situation in the human. In contrast, a prior art method that uses an albumin promoter to direct expression of inappropriately large amounts of CYP3A4 will distort the role of this protein and give misleading results. For instance, the drug might in fact only be a weak substrate for CYP3A4, but will nevertheless be metabolised aggressively if high amounts of the protein are present.

As a result, the human protein is produced in the correct place at the correct time. This imparts a realism to the model that is simply not possessed in the prior art systems.

### Preferred constructs

In some circumstances; the target gene and the human incorporated gene may share a leader sequence. This may be achieved by retaining at least one intron in the construct, which usually results in a better expression. This strategy also ensures that the gene product will be guided to the right intracellular location. The human leader sequence might be able to fulfil that function as well, but it is often safer to use the mouse leader instead. For instance, in the case of MRP2, the "leader sequence" of the mouse protein, which is encoded by exon 1, may be retained. The human cDNA without sequences from exon 1 is then introduced into exon 2 of the mouse genomic sequence. The original splice sites for mouse intron 1 will be retained, so that this construct encodes a fusion protein of amino acids from mouse exon 1 and human exons 2-32. This construct ensures a high level of expression and also that the MRP2 is guided to its correct location, the plasma membrane.

In other cases, the incorporated human gene may be brought under control of the promoter of the appropriate target animal gene. For example, in the case of MDR1, the cDNA of human MDR1 may be fused to the translational start site of the corresponding mouse genes (*Mdr1a* or *Mdr1b*). In the example of PXR, a hybrid of human PXR cDNA and genomic sequences may be fused to the translational start site of the mouse PXR gene, whereby the mouse Start-ATG is retained. In the case of CAR, the human sequence may be fused to the translational start site of the mouse CAR gene.

### Transgenic Mice

In preferred embodiments of the present invention as described above and below, the transgenic non-human animal and tissues or cells derived therefrom is a mouse.

Although the use of transgenic animals poses questions of an ethical nature, the benefit to man from studies of the types described herein is considered vastly to outweigh any suffering that might be imposed in the creation and testing of transgenic animals. As will be evident to those of skill in the art, drug therapies require animal testing before clinical trials can commence in humans and under current regulations and with currently available model systems, animal testing cannot be dispensed with.

The invention aims to reduce the extent of attrition in drug discovery. Whenever a drug fails at a late stage in testing, all of the animal experiments will in a sense have been wasted. Stopping drugs failing therefore saves test animals' lives. Therefore, although the present invention relates to transgenic animal the use of such animals should reduce the number of animals that must be used in drug testing programmes.

An advantage of the present invention is that it avoids problems of species divergence between the human and other mammals that have conventionally been used as test models. One example is provided by the family of peroxisome proliferator activated receptors (PPARs), to which various drugs were in the past developed as hypolipidaemia agents. The development of these drugs was stopped, as they were identified in mouse models to be epigenetic carcinogens. It eventually turned out that that the difference in toxicity between species could be attributed to differences in levels of PPARα in the liver. The phenomenon apparent in the mouse does not occur in humans, because of lower levels of PPARα protein that are present in the liver. There are very clear advantages to models that exhibit *bona fide* levels of protein expression that reflect those present in the human body.

Preferably, the human DNA sequences are each independently linked to human or non-human animal regulatory DNA sequences (e.g. the endogenous human or non-human promoter). This linkage is distinct from the prior art and provides the advantage of improvement, over prior art models as this further advances the mirroring of an in vivo human situation.

A particular advantage of the humanised transgenic mice, cells and tissues, of the present invention is that they combine the benefits of normal experimental animal models with those of human cell or tissue culture in a single system. This system or humanised transgenic animal will provide the pharmaceutical industry with an improved alternative for use in all pre-clinical metabolism, toxicity and drug disposition studies.

### Cells

Another aspect of the invention relates to cells, modified so as to possess properties according to any one of the above-described aspect of the invention. Hepatocytes and neuronal cells are preferred cell types according to the present invention. The cells are mouse cells.

Cells according to this aspect of the invention may be created from transgenic mice according to the invention using standard techniques, as will be clear to the skilled readar**,** imbued with knowledge of the present invention. Suitable methods are described in many standard laboratory manuals, such as Davis et al., Basic Methods in Molecular Biology (1986); Sambrook Molecular Cloning; A Laboratory Manual, Third Edition (2000); Ausubel et al., 1991 [supra]; Spector, Goldman & Leinwald, 1998).

In one aspect, such cells may be mouse cells, generated according to any one of the above-described aspects of the invention. One preferred method of generating such cells is to cross a humanised mouse, as described above, with SV40 immortalised mouse (for example, the immorta-mouse (Taconix). Cells may subsequently be isolated from such animals according to well known techniques in the art. In contrast to prior art transgenic systems, which used the albumin promoter that is only active in the liver and thus only able to generate hepatocytes, cells from transgenic mice generated according to the present invention may be of a diverse selection of different cell types, including cells of significant importance to pharmacokinetics analyses, such as hepatocytes and neuronal cells.

Stem cells isolated from transgenic mice according to the invention, with properties as described above are also useful aspects of the present invention. Such cells may be pluripotent, or partially differentiated. Stem cells may be adult stem cells or embryonic stem cells. More generally, stem cells employed may be from a post-embryonic developmental stage e.g. foetal, neonatal, juvenile, or adult. Stem cells isolated in this manner may be used to generate specific types of cells such as hepatocytes and neuronal cells. Such cells also form an aspect of the present invention.

### Preferred selections of replaced genes

The invention also describes a mouse, tissue or cells derived therefrom incorporating:
(i) at least one human DNA sequence encoding a transcription factor; and
(ii) at least one human DNA sequences encoding a drug transporter protein;
and whose endogenous equivalent genes have been annulled.

The inventors are of the view that in the case of models of drug metabolism, it is important to generate mice that are not just transgenic for particular drug metabolising enzymes, but also to incorporate in these models, proteins that are transporters of drugs i.e. drug transporter proteins. For example, many compounds that activate PXR, the nuclear transcription factor, are also substrates for MDR1 and are thus transported out of cells by this protein. Therefore, in order to create a faithful model of the *in vivo* situation, mice must preferably be transgenic for drug transporter proteins, otherwise a misleading impression will be obtained of the intracellular effects of any particular concentration of drug. Of the drug transporter proteins, MDR1 is preferred. In the case of prior art transgenic mice models, of course, the redundancy of drug transporters that is generated by the presence of both *mdr1a* and *mdr1b* can generate misleading results and so preferably, both these mouse genes should be knocked out and replaced with the human *mdr1* gene.

The expression of genes encoding drug transporter proteins such as MDR1 is also activated by the PXR-based signalling system. Accordingly, because the expression of the phase I, phase II and drug transporter genes is linked by PXR, in addition to the fact that the products of these genes have varied effects on the levels and metabolism of drugs and their metabolites, the integrity of co-ordinated regulation that is maintained according to the present invention is extremely advantageous, particularly when compared to prior art systems.

In addition, MDR is expressed at a significant degree in the gastro-intestinal (GI) tract and in the environment of the blood-brain barrier. Since both the GI tract and the blood brain harder are significant sources of drug transport into the blood stream, the presence of physiologically relevant MDR expression levels imparts an important aspect of the drug metabolism process to the drug model and in demonstrating pharmacological activity. The presence of MDR in the GI tract, for example, can render orally-delivered drug not bioavailable. MDR is very important for drug transport both in an out of the brain. MDR also transport drugs from somatic cells in the liver into the bile.

Other preferred combinations of drug metabolism genes are listed as follows.

The invention also describes a mouse tissue or cells derived therefrom incorporating:
(i) at least one human DNA sequence encoding a transcription factor; and
(ii) at least one human DNA sequence encoding a phase-1 drug-metabolising enzyme; and/or
(iii) at least one human DNA sequence encoding a drug transporter protein;
and whose endogenous equivalent genes have been annulled.

The invention also describes a mouse tissue or cells derived therefrom incorporating:
(i) at least one human DNA sequence encoding a transcription factor; and
(ii) at least one human DNA sequence encoding a phase-2 drug-metabolising enzyme; and/or
(iii) at least one human DNA sequence encoding a drug transporter protein;
and whose endogenous equivalent genes have been annulled.

The invention also describes a mouse tissue or cells derived therefrom incorporating:
(i) at least one human DNA sequence encoding a transcription factor;
(ii) at least one human DNA sequence encoding a phase-1 drug-metabolising enzyme;
(iii) at least one human DNA sequence encoding a phase-2 drug-metabolising enzyme; and
(iv) at least one human DNA sequence encoding a drug transporter protein,
and whose endogenous equivalent genes have been annulled.

Preferably, this aspect includes any one or more of the features hereinbefore described.

The invention also describes a mouse tissue or cells derived therefrom incorporating human DNA sequences encoding a PXR and a CAR transcription factor and at least one human DNA sequence encoding a phase-1 drug-metabolising enzyme and at least one human DNA sequence encoding a phase-2 drug-metabolising enzyme and at least one human DNA sequence encoding a drug transporter protein, wherein the non-human animal, tissue or cells from which the endogenous equivalent genes have been annulled.

The invention also describes a mouse tissue or cells derived therefrom incorporating:
(i) a human DNA sequence encoding a PXR and/or a CAR transcription factor;
(ii) a human DNA sequence encoding the CYP3A4 enzyme; and
(iii) a human DNA sequence encoding the MDR1 protein.

This aspect of the invention is of particular utility in that the CYP3A4 enzyme is of particular importance in the GI tract and therefore an animal humanised for this isoform will be of importance in investigating the role of drug metabolism in drug bioavailability in the gut. In addition, as set out above, the MDR1 protein is known to be expressed at the blood-brain barrier and when incorporated into the above described humanised mouse will provide a truly representative scenario of the uptake of drugs/xenobiotics into and out of the brain in man.

The invention also describes a mouse, tissue or cells derived therefrom incorporating:
(i) a human DNA sequence encoding a PXR and/or a CAR transcription factor;
(ii) a human DNA sequence encoding the CYP3A4 enzyme;
(iii) a human DNA sequence encoding the UGT1A enzyme; and
(iv) a human DNA sequence encoding the MDR1 protein.

Other preferred combinations include combinations of a PXR and/or a CAR transcription factor, a human DNA sequence encoding the MDR1 protein and one or more of: CYP2A9, the CYP3A-cluster, the CYP2C-cluster and UGT or the UGT0-cluster.

### Methods for introducing replacement genes

According to a further aspect of the invention there is provided a method of introducing at least one functional human transcription factor into a mouse cell whose own endogenous equivalent gene(s) expressing the aforesaid protein have been rendered inactive, the mouse cell derived therefrom further incorporating at least one or more of the following further human DNA sequences selected from the group comprising:
(i) a DNA sequence encoding a phase-1 drug metabolising enzyme;
(ii) a DNA sequence encoding a phase-2 drug-metabolising enzyme; and/or
(iii) a DNA sequence encoding a drug transporter protein
and whose own endogenous equivalent genes expressing the aforesaid proteins have been rendered inactive;
the method comprising introducing DNAs encoding said human transcription factor, phase-1 and 2 drug metabolising enzyme and/or drug transporter protein such that said human expression products remain functional where the mouse cell's own endogenous genes are rendered inactive, and
wherein the mouse genes whose protein products are analogous to the protein products of the introduced human DNA sequences are deleted by either direct targeting with their human counterparts or by flanking these genes or gene clusters with recognition sites and subsequent recombinase-mediated deletion, such that the human DNA sequence is inserted at the point in the genome where the endogenous equivalent gene naturally occurs; and
wherein the cell is humanised for both PXR and CAR alone or in combination.

According to the present invention endogenous non-human genes whose protein products are analogous to the protein products of the introduced human DNA sequences are deleted. This can be done preferably either by direct targeting with their human counterparts or by flanking these genes or gene clusters with recognition sites and subsequent recombinase-mediated deletion.

According to the present invention, genes that are inserted into the transgenic model are inserted at the point in the genome where the endogenous equivalent gene or gene cluster naturally occurs. This has the advantage that the context of the gene locus is retained which means that the fidelity of transcription from this site is as close as possible to the level of transcription that occurs in the wild type system.

Furthermore, according to the invention, the endogenous equivalent gene or genes to those that are inserted into the transgenic system have been annulled. "Annulled" is meant to include silencing or deletion or rendering inactive so that the non-human animal's endogenous equivalent gene is unable to express the gene product(s). A preferable way in which to annul the expression of the endogenous equivalent gene or genes and simultaneously to insert the replacement gene at the point at which it naturally occurs is by the process of homologous recombination, described above. According to this methodology, homology arms of sequence complementary to sites in the target genome flank the insertion sequence and these are used to direct insertion of the desired human gene or genes.

For example, in the case of MDR1, the drug transporter protein, different animal species have different MDR1 isotypes and expression profiles. The mouse has two genes encoding active drug transporters (MDR1a and MDR1b), whose tissue expression is mutually exclusive. In contrast, MDR1 is the only functioning drug transporter of the two MDR genes present in the human (MDR1 and MDR3). When creating a mouse transgenic for MDR, therefore, MDR1 should preferably replace both the endogenous MDR genes, MDR1a and MDR1b.

Another example can be provided by the case of C'YP2D6, where there are nine genes in the mouse, corresponding to only one functional gene in humans. Replacement of the mouse gene cluster with the human gene is relatively simple though, since the latter spans less than around 40kb of genomic sequence.

DNA sequences may be deleted by, for example, Cre/lox-mediated deletions. This type of deletion is suitable for deleting of large fragments of DNA (200kb to several megabases). The method has been described in the following papers (Li ZW, Stark G, Gotz J, Rulicke T, Gschwind M, Huber G, Muller U, Weissmann C. Generation of mice with a 200-kb amyloid precursor protein gene deletion by Cre recombinase-mediated site-specific recombination in embryonic stem cells Proc Natl Acad Sci U S A. 1996 Jun 11;93(12):6158-62. Erratum in: Proc Natl Acad Sci U S A 1996 Oct 15;93(21):12052 and in Su H, Wang X, Bradley A. Nested chromosomal deletions induced with retroviral vectors in mice. Nat Genet. 2000 Jan;24(1):92-5).

Cre/lox-mediated insertions of large fragments may also be used to insert the human DNA sequences by a method described in Call LM, Moore CS, Stetten. G, Gearhart JD. A cre-lox recombination system for the targeted integration of circular yeast artificial chromosomes into embryonic stem cells. Hum Mol Genet. 2000 Jul 22;9(12):1745-51.

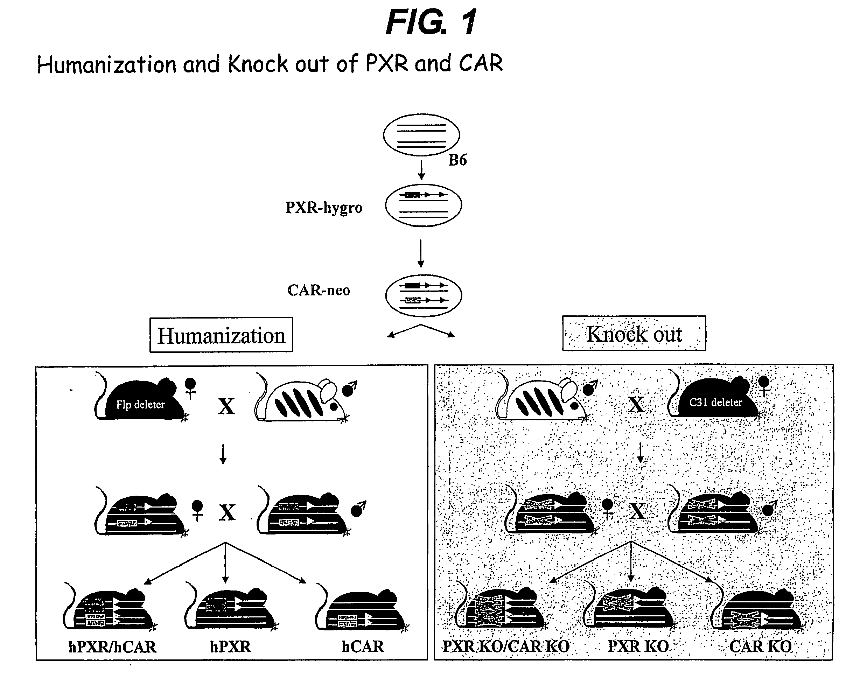

Preferably, the transgenic mouse is humanised for a drug transporter protein by the hitherto undisclosed "knock-in" approach, as shown in the schematic representation of accompanying Figures 1, 2 and 3.

The transgenic mouse of the present invention is humanised for both PXR and CAR alone or in combination, more preferably in combination.

Preferably, the human genes are at least partially conserved within a construct.

Preferably, in the case of a transgenic mouse expressing the human PXR gene, the PXR construct retains the intron-exon structure between exons 4 and 6. This advantageously retains the sequence where most splice variants are observed and is conveniently located within the ligand-binding domain. The preferred human PXR sequence therefore contains the cDNA of exons 1-4, the genomic sequences of intron 4, exon 5 and intron 5 and the cDNA for exons 6-9.

It will be appreciated that alternative splice products between these exons, which might account for proteins with distinct ligand-binding capacity, will be covered by the constructs of the present invention.

Preferably, the CAR construct retains the intron-exon structure between exons 2 and 9. This advantageously retains complete genomic structure within the targeting vector and permits coverage of all splice variants of human CAR.

Other preferred constructs are described above.

In one embodiment of the invention the transgenic mouse are produced *de novo* so as to include all the aforementioned features by methods wherein, for example, cre/lox mediated deletion of large fragments of DNA (200kb to several megabase) are achieved (Li ZW, et al. Generation of mice with a 200-kb amyloid precursor protein gene deletion by Cre recombinase-mediated site-specific recombination in embryonic stem cells Proc Natl Acad Sci USA. 1996 Jun 11;93(12):6158-62 Erratum in: Proc Natl Acad Sci U S A 1996 Oct 15;93(21):12052 and in Su H et al Nested chromosomal deletions induced with retroviral vectors in mice. Nat Genet. 2000 Jan;24(1):92-5), and where cre/lox-mediated insertions of large fragments are achieved as described in Call et al. A cre-lox recombination system for the targeted integration of circular yeast artificial chromosomes into embryonic stem cells. Hum Mol Genet. 2000 Jul 22;9(12):1745-51.

In another embodiment of the invention the transgenic mouse of the present invention is produced by crossing. For example, the animal of WO2004/007708 might be crossed with those transgenic animals that have been humanised with PXR and /or CAR and include further modifications with respect to phase-2 drug metabolising enzymes and drug transporter protein in either transgenic strain of the mouse.

In a further embodiment of the invention the transgenic mouse is produced *de novo* so as to include all of the aforementioned features, by the methods as hereinafter disclosed.

It will be appreciated that ideally all of the introduced human DNAs are substantially under the control of human promoters.

### Model systems

Advantageously, the present invention provides a transgenic mouse that mimics the human mechanisms of metabolism, disposition or toxicity of drugs or other xenobiotic compounds on a mouse cell by introducing into a mouse cell one or more human DNA sequences comprising coding and regulatory sequences necessary to reproduce the regulation and function of one or more proteins responsible for human metabolism, disposition or toxicity of drugs or other xenobiotic compounds where the mouse cell has undergone deletion of endogenous genes encoding proteins whose functions are analogous to those encoded by the introduced human DNA sequences so that the mouse cell can be used as a model system for determining the metabolism, disposition or toxicity of drugs or other xenobiotic compounds in a homologous human cell.

It is possible, according to the invention, to "personalise" a particular transgenic system to suit a phenomenon that is worthy of investigation. For example, CYP3A4 gene expression levels may vary as much as 60 times between individuals, and in any individual may also vary over time. This is partly because of inherent genetic differences, but more importantly due to variability in exposure to drugs, toxins, food products and other environmental variables. The system is an adaptive response system which will only keep high enzyme levels for as long as they are needed. Any other implementation would be wasteful. This means that it is not generally appropriate, in any test system, merely to test the effect of a particular drug concentration at one level of GYP3A4. The effects of the drug must ideally be assessed at high CYP3A4 levels and also at low CYP3A4 levels so that response is tested in both a high and a low P450 environment Of course, systems according to the prior art, using the albumin promoter, simply cannot reflect the real situation found *in vivo.*

Another example is provided by the CYP2D6 protein, which plays a major role in the metabolism of neuroleptic drugs (e.g. anti-depressants and drugs used for treatment of schizophrenia), and is thus of significant importance. Pharmaceutical companies are reluctant to back a drug that is metabolised by CYP2D6 and therefore need to know as soon as possible during development of any drug, whether or not it has a CYP2D6 liability. This gene shows variation between individuals, and in fact expression is absent in around 6% of Caucasian individuals. It would be of immense benefit to be able to study the metabolism of drugs, particularly of anti-depressants, in environments of both high and low CYP2D6 levels.

These advantages also allow carcinogenicity testing to be performed as well as the acute pharmacological tests described above. For example, according to the invention, P450 levels, either as single genes or a multiple genes and preferably as clusters of genes, may be raised to artificially high levels in order to test for potentially carcinogenic effects of metabolites. At physiologically normal levels, the effects of such metabolites might not be evident. The marked species difference in carcinogenicity of compounds between rodents and man result in the main from the different rates of generation of toxic or mutagenic metabolites, along with other differences in pharmacokinetics and distribution. The ability to increase gene levels in entire clusters is important as it retains the substrate crosstalk between the different proteins expressed by the genes in the cluster.

Bespoke systems of this type may also be exploited to investigate disease. An example is provided by Gilbert syndrome, a phenomenon caused by a polymorphism in the UGT1A1 gene implicated in drug metabolism. According to the invention, a transgenic model animal may incorporate the polymorphism-containing gene in order to allow this syndrome to be evaluated.

According to a yet further aspect of the invention there is described a host cell transfected with a nucleic acid constcuct(s) according to any one of the previous aspects of the invention.

According to a yet further aspect of the invention, there is described a transgenic mouse in which the cells of the animal express the protein(s) encoded by the nucleic acid construct(s) according to any one of the previous aspects of the invention. The transgenic animal is a mouse.

In embodiments of the invention relating to the preparation of a transgenic host cell or a transgenic mouse comprising the use of a nucleic acid construct as previously described, the cell or mouse may be subjected to further transgenesis, in which the transgenesis is the introduction of an additional gene or genes or protein-encoding nucleic acid sequence or sequences. The transgenesis may be transient or stable transfection of a cell or a cell line, an episomal expression system in a cell or a cell line, or preparation of a transgenic non-human animal by pronuclear microinjection, through recombination events in non-embryonic stem (ES) cells, random transgenesis in non-human embryonic stems (ES) cells or by transfection of a cell whose nucleus is to be used as a donor nucleus in a nuclear transfer cloning procedure.

Methods of preparing a transgenic cell or cell line, or a transgenic mouse, in which the method comprises transient or stable transfection of a cell or a cell line, expression of an episomal expression system in a cell or cell line, or pronuclear microinjection, recombination events in ES cells, or other cell line or by transfection of a cell line which may be differentiated down different developmental pathways and whose nucleus is to be used as the donor for nuclear transfer; wherein expression of an additional nucleic acid sequence or construct is used to screen for transfection or transgenesis in accordance with the previous aspects of the invention. Examples include use of selectable markers conferring resistance to antibiotics added to the growth medium of cells, for instance neomycin resistance marker conferring resistance to G418. Further examples involve detection using nucleic acid sequences that are of complementary sequence and which will hybridise with, or a component of, the nucleic acid sequence in accordance with the previous aspects of the invention. Examples would include Southern blot analysis, northern blot analysis and PCR.

Mouse cell or animals produced by the method of the invention can be used as model systems for determining the metabolism of drugs or other xenobiotic compounds in a human.

According to a yet further aspect of the invention there is describes the use of a transgenic mouse, tissues and/or cells derived therefrom as hereinbefore described that have been modified to contain and express DNA encoding at least one functional human transcription factor, at least one phase-1 drug metabolising enzyme, at least one phase-2 drug-metabolising enzyme and at least one drug transporter protein so as to investigate xenobiotic metabolism or toxicity in the transgenic mouse, tissues and/or cells derived therefrom or other properties or functions of the introduced human proteins such as metabolism and/or biosynthesis of endogenous compounds.

The system of the present invention allows function and regulation of human mechanisms of xenobiotic metabolism, disposition and toxicity to be studied in any tissue or cell type, for instance gastrointestinal tract, blood-brain barrier, liver, kidney in a single animal, tissue or cell derived therefrom.

The system of the present invention may be applied to study effects of human metabolism, disposition or toxicity on anti-tumour effects of a drug in an animal xenograft experiment by expressing humanised metabolic pathways in a non-human grafted tumour cell line and/or in the host animal.

### Reporters

The present invention also, advantageously describes mouse cells and transgenic mice incorporating introduced reporter genes so that such cells or animals can be used to determine indications of pathways of metabolism of drugs or other xenobiotic compounds in a human cell by convenient assay of the products of reporter gene expression.

Where reporter genes have been incorporated in mouse cell or transgenic mice produced by the method of the invention (see below), the cells or animals can be used to determine regulation of genes and also give indications of the likely mechanism and metabolism of drugs or other xenobiotic compounds in an homologous human cell by assaying expression of the reporter gene DNA sequence. The cells or animals can also be used to give indications of the extent of metabolism of drugs or other xenobiotic compounds. For example, analysis of the distribution of reporter gene expression within any particular tissue allows the extent of induction of gene expression to be monitored in response to a particular drug compound.

According to a further aspect of the invention there is described a non-human animal, tissue or cells derived therefrom incorporating a promoter linked transcriptionally to a human DNA sequence encoding
(i) a transcription factor; and
(ii) a DNA sequence encoding a phase-1 drug-metabolising enzyme; and/or
(iii) a DNA sequence encoding a phase-2 drug-metabolising enzyme; and/or
(iv) a DNA sequence encoding a drug transporter protein.

The promoter of the transcription factor and/or phase 1 and/or phase 2 drug-metabolizing enzyme and/or drug transporter protein may thus be linked to a reporter which allows monitoring for the relative regulation of at least one enzyme involved in drug/xenobiotic disposition. Such an embodiment advantageously allows not only for relative regulation of the enzymes but regulation in both a tissue-specific manner in the transgenic animal or in the whole animal itself in a non-invasive manner as well as the extent and potency of gene induction. Reporters may be linked to the promoters of two or more of (i), (ii), (iii) or (iv) listed above.

Reporter genes are nucleic acid sequences encoding directly or indirectly assayable proteins. They are used to replace other coding regions whose protein products are unsuitable or not amenable to the assay envisaged. Suitable reporter genes that are known in the art and may be used in the present invention are selected from those genes encoding proteins including but not limited to: chloramphenicol-acetyltransferase, β-galactosidase, β-glucuronidase, luciferase, beta-galactosidase, green fluorescent protein, secreted alkaline phosphatase (SEAP), major urinary protein (MUP) or human chorionic gonadotrophin (hCG). It will be understood that the above list of suitable reporter genes is not exhaustive or exclusive and is not intended to limit the scope of the application. The skilled artisan may select another reporter system which will equally be applicable to the present invention.

According to the invention, the promoters that are preferred targets for linkage to reporter genes are PXR, CAR, CYP3A4, CYP2C9, CYP2C19, CYP2B6, CYP2B6, UGT1A, MRP2 and MDR1.

Animals, tissues and cells according to these aspects of the invention allow very useful elements of the process of gene regulation in drug metabolism to be elucidated. For example, although PXR is known to be of great significance in the transcriptional control of the CYP3A4 gene, there are likely to be other important active mechanisms. Exposing a CYP3A4 reporter animal or cell to a drug compound in either a humanised PXR or a PXR null background would be useful in exploring other ways of regulating CYP3A4 than by way of PXR. Another case of interest would be to expose an *mdr1* reporter animal or cell to a drug compound in both a humanised PXR and PXR null background to detect aspects of *mdr1* expression that are independent of PXR regulation.

Accordingly, mouse animals, tissues and cells according to this aspect of the invention may comprise a promoter linked transcriptionally to a human DNA sequence encoding a phase-1 drug-metabolising enzyme; a DNA sequence encoding a phase-2 drug-metabolising enzyme; and/or a DNA sequence encoding a drug transporter protein in a null background for PXR, CAR or any other transcription factor. By "null background" is meant that the gene or genes have been annulled, according to the definition provided above. Such animals, cells, or tissues might be compared under similar conditions (for example, in the presence and absence of a drug or drugs) to in a humanised PXR or CAR background. Accordingly, animals, tissues and cells according to this aspect of the invention may comprise a promoter linked transcriptionally to a human DNA sequence encoding a phase-1 drug-metabolising enzyme; a DNA sequence encoding a phase-2 drug-metabolising enzyme; and/or a DNA sequence encoding a drug transporter protein in a humanized background for a transcription factor. Such a transcription factor may be PXR alone, CAR alone, PXR and CAR or any other single transcription factor or combination of transcription factors described herein.

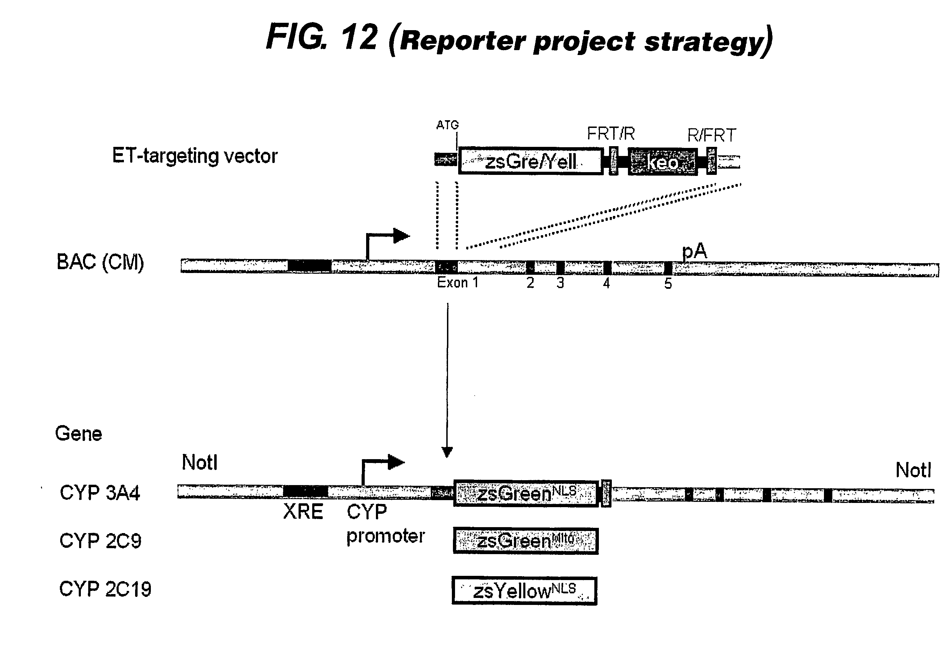

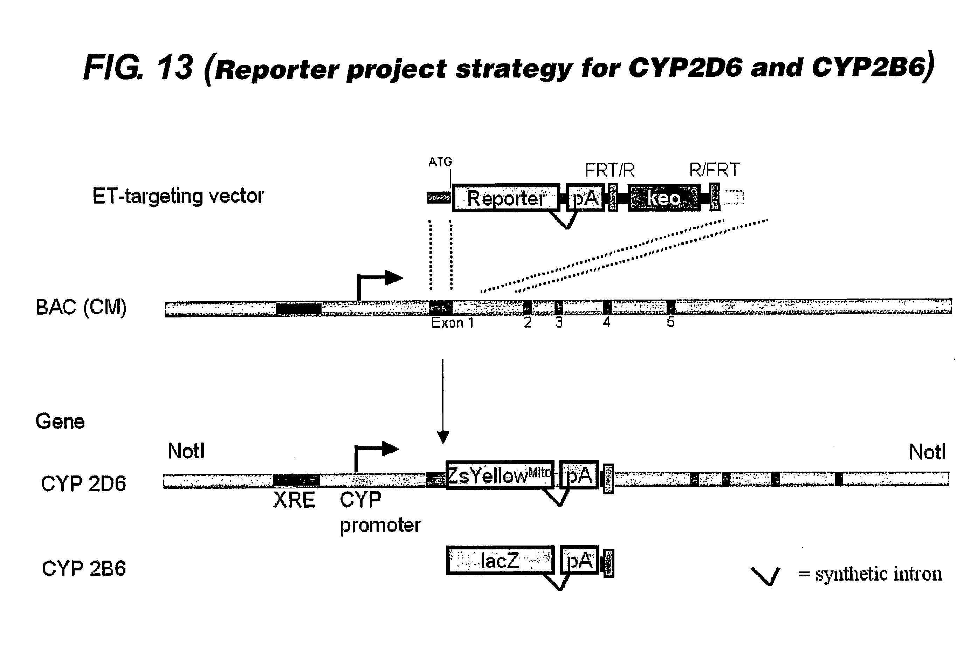

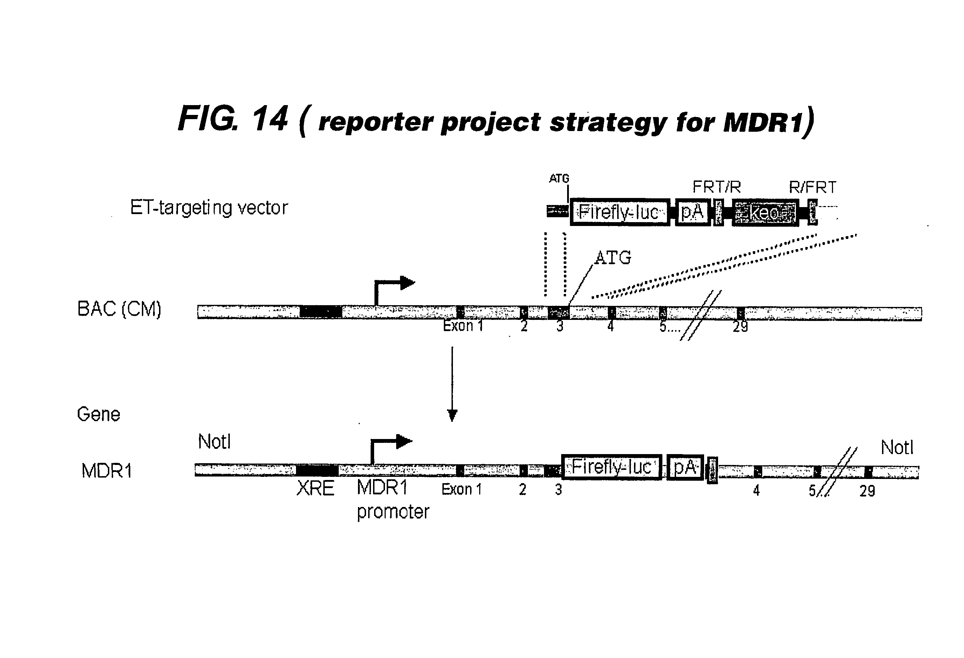

By "linked transcriptionally" is meant that the activity of the promoter dictates the expression level of the reporter protein. Preferably, a reporter gene is fused to the translational start site of the corresponding human gene whose promoter is to be investigated. In the case of the CYP3A4, CYP2C9 and CYP2C19 promoters, the transcript of the reporter gene may not be terminated by a polyA motif, but the constructs are designed such that the endogenous polyA motif is potentially used These constructs are therefore dependent on a correct splicing of the exons 3' to the reporter (see Figure 12). In the cases of CYP2D6 and CYP2B6, the transcript of the reporter gene is preferably terminated by a polyA motif linked to the reporter gene with a synthetic intron (see Figure 13). In case of MDR1, the transcript of the reporter gene is preferably terminated by a polyA motif without an additional intron (see Figure 14).

According to a further aspect of the invention there is described a mouse, tissue or cells derived therefrom incorporating at least one human DNA sequence encoding at least one transcription factor under control of a transcription factor promoter and whose endogenous equivalent genes have optionally been annulled, the non-human animal, tissue or cells further incorporating a promoter linked transcriptionally to a human DNA sequence encoding:
(i) a DNA sequence encoding a phase-1 drug-metabolising enzyme; and/or
(ii) a DNA sequence encoding a phase-2 drug-metabolising enzyme; and/or
(iii) a DNA sequence encoding a drug transporter protein.

The mouse, and tissues or cells of this aspect of the invention may incorporate a promoter linked transcriptionally to a DNA sequence encoding a phase-1 drug-metabolising enzyme and a DNA sequence encoding a phase-2 drug-metabolising enzyme. The non-human animal, and tissues or cells may incorporate a promoter linked transcriptionally to a DNA sequence encoding a phase-1 drug-metabolizing enzyme and a DNA sequence encoding a drug transporter protein. The non-human animal, and tissues or cells may incorporate a promoter linked transcriptionally to a DNA sequence encoding a phase-2 drug-metabolising enzyme and a DNA sequence encoding a drug transporter protein. Examples of suitable phase-1 drug-metabolising enzymes, phase-2 drug-metabolising enzymes and drug transporter proteins are described herein.

In another aspect, for example, the promoter activity of the *mdr1* gene may be investigated. In this scenario, animals, tissues and cells may comprise a promoter linked transcriptionally to a human DNA sequence encoring a transcription factor, a promoter linked transcriptionally to a human DNA sequence encoding a phase-1 drug-metabolising enzyme; and/or a promoter linked transcriptionally to a DNA sequence encoding a phase-2 drug metabolising, enzyme in a humanised or null background for *mdr1.*

According to this aspect of the invention there is described a mouse, tissue or cells derived therefrom incorporating at least one human DNA sequence encoding at least one drug transporter protein under control of a drug transporter promoter and whose endogenous equivalent genes have been annulled, the non-human animal, tissue or cells further incorporating a promoter linked transcriptionally to a human DNA sequence encoding:
(i) a DNA sequence encoding a transcription factor; and/or
(ii) a DNA sequence encoding a phase-1 drug-metabolising enzyme; and/or
(iii) a DNA sequence encoding a phase-2 drug-metabolising enzyme.

The mouse, and tissues or cells of this aspect of the invention may incorporate a promoter linked transcriptionally to a DNA sequence encoding a phase-1 drug-metabolising enzyme and a DNA sequence encoding a phase-2 drug-metabolising enzyme. The mouse, and tissues or cells may incorporate a promoter linked transcriptionally to a DNA sequence encoding a phase-1 drug-metabolising enzyme and a DNA sequence encoding a transcription factor. The non-human animal, and tissues or cells may incorporate a promoter linked transcriptionally to a DNA sequence encoding a phase-2 drug-metabolising enzyme and a DNA sequence encoding a transcription factor. Examples of suitable phase-1 drug-metabolising enzymes, phase-2 drug-metabolising enzymes and transcription factor proteins are described herein.

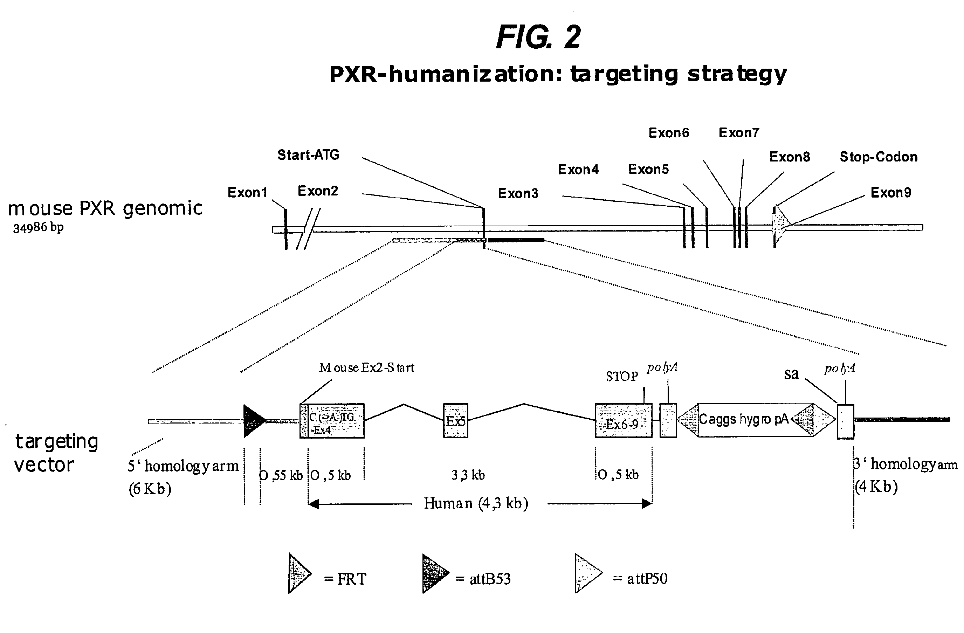

According to a yet further aspect of the invention, there is described a nucleic acid construct comprising a targeting vector substantially as depicted in Figure 2, Figure 3, Figure 12, Figure 13 and/or Figure 14.

Preferably, the construct further includes for the humanisation and corresponding knock-out of at least one phase-1 drug metabolising enzyme, at least one phase-2 drug-metabolising enzyme and at least one drug transporter protein in either the same construct or further independent constructs.

### Cells

In another aspect of the invention, a mouse cell is produced by any one of the above-described aspects of the invention. In preferred aspects, at least one human regulator DNA sequence associated with the gene encoding a protein responsible for determining the human metabolism, disposition, distribution or toxicity of drugs or other xenobiotic compounds is operatively linked to a DNA sequence whose expression can conveniently be measured by assay of transcription or translation products to produce a reporter gene DNA sequence which is introduced into the non-human animal cell. This embodiment provides linkage with one or more reporter sequences such as human chorionic gonadotrophin (hCG).

### Assays

The mice, tissues and cells of the present invention may be used to determine how a drug compound is metabolised by a human. In particular, it is possible to examine whether a drug compound modulates the activity or expression levels of a transcription factor, a drug metabolising enzyme or a drug transporter protein. It is possible to examine the ratio of the levels of activation or expression of a transcription factor, a drug metabolising, enzyme or a drug transporter protein induced by a drug compound. It is possible to examine whether a drug compound influences the disposition or distribution of a transcription factor, a drug metabolising enzyme or a drug transporter protein within the tissues of the body. It is possible to examine whether a drug compound influences the duration of expression of a transcription factor, a drug metabolising enzyme for a drug transporter protein.

It is possible to measure a phenotypic change in the animal, such as a physiological effect. Such a physiological effect may be, for example, a disease condition (such as biliary necrosis) or a toxic side-effect.

It is possible to examine the rate of metabolism of a drug compound. The rate of metabolism may be determined by measuring the toxicity or activity mediated by the administration of the compound, measuring the half-life of the compound, or measuring the level of a drug metabolising enzyme. For example, the rate of metabolism of the compound may be measured as the rate of formation of the oxidize product or the formation of a subsequent product generated from the oxidized intermediate. Alternatively, the rate of metabolism may be represented as the half-life or rate of disappearance of the initial compound or as the change in toxicity or activity of the initial compound or a metabolite generated from the initial compound. The half-life may be measured by determining the amount of the drug compound present in samples taken at various time points. The amount of the drug compound may be quantified using standard methods such as high-performance liquid chromatography, mass spectrometry, western blot analysis using compound specific antibodies, or any other appropriate method.

It is also possible to examine whether a drug compound is metabolised to a toxic or carcinogenic metabolite, for example, by measuring its covalent binding to tissues, proteins or DNA or by measuring glutathione depletion.

Preferably, measurements of the type described above are performed at more than 1, 3, 5,10 or more time points after administration of the drug compound.

Accordingly, further aspects of the invention relate to screening methods that are provided to determine the effect of a drug compound on the activity or expression level of a transcription factor, a drug metabolising enzyme or a drug transporter protein. Such methods involve administering a drug compound to a transgenic mouse according to any one of the aspects of the invention described above, or a tissue or cell derived therefrom.

The screening step may involve measuring the induction of a gene coding for a transcription factor, a drug metabolising enzyme or a drug transporter protein. The screening step may involve measuring the level of expression of a transcription factor, a drug metabolising enzyme or a drug transporter protein or the duration of such expression. The screening step may involve measuring the distribution of expression of a transcription factor, a drug metabolising enzyme or a drug transporter protein.

The assay can be performed in the presence and absence of the drug compound to ascertain differences in distribution, metabolism and toxicity. The effects of the drug compound in the presence and absence of a particular gene or genes can be ascertained by evaluating the effects of the drug compound on different transgenic animals, cells or tissues. For example, the effects of the drug compound could be evaluated between an animal with a null background and an animal humanised for the gene or genes of interest (e.g. PXR, CAR, MDR1, a phase I metabolising enzyme or a phase 2 metabolising enzyme).

More than one drug compound may be administered. For example, a drug compound is determined to activate the CAR transcription factor if the compound mediates induction of the CAR gene. A CAR receptor inverse agonist such as clotrimazole can also administered to an animal expressing the human CAR receptor as a control. Assays according to further aspects of the invention may provide a screening method for determining whether the metabolism of a first drug compound is modulated by a second drug compound. This method involves administering the first compound in the presence and absence of the second compound to a transgenic mouse according to any one of the above-described aspects of the invention, or a tissue or cell derived therefrom, and monitoring for a phenotypic effect. Alternatively, as above, the screening step may involve measuring the induction of a gene, the level, duration or distribution of expression, of a transcription factor, a drug metabolising enzyme or a drug transporter protein. The second compound is determined to modulate the metabolism of the first compound if the second compound effects a change in any one of these tested factors. For example, a physiological effect may be assayed by measuring the toxicity or activity mediated by the administration of the first compound or measuring the half-life of the first drug compound.

In this manner, assays may be used to facilitate the identification of analogs of a drug compound that have reduced or undetectable ability to activate or induce expression of a particular protein, and thus are expected to have fewer side-effects or a longer half-life *in vivo.*

The invention will now be described with reference to the following figures wherein:
Figure 1 shows a schematic representation of humanisation and knock out of PXR/CAR;
Figure 2 shows a schematic representation of a composite cDNA with genomic sequences for the PXR humanisation strategy;
Figure 3 shows a schematic representation of a composite cDNA with genomic sequences for the CAR humanisation strategy;
Figure 4 shows mouse *mdr1a* targeting vector;
Figure 5 shows mouse *mdr1b* targeting vector;
Figure 6 shows construct for MRP2 humanisation;
Figure 7 shows targeting strategy for PXR humanisation;
Figure 8 shows targeting strategy for CAR humanisation;
Figure 9 shows strategy for CYP3A4 humanisation;
Figure 10 shows strategy for CYP2C9 humanisation;
Figure 11 shows overall strategy for cluster exchange;
Figure 12 shows overall strategy for reporter constructs;
Figure 13 shows reporter project strategy for CYP2D6 and CYP2B6;
Figure 14 shows reporter project strategy for MDR1;
Figure 15 shows an example of a PXR typing PCR;
Figure 16 shows Taqman typing of wild type and transgenic mice. All probes and primers used were pre-optimised TaqMan^{®} Genomic Assay kits, which were purchased from Applied Biosystems. All assays used were inventoried by Applied Biosystems. Shown are the results from TaqMan analysis of liver (A, B) and small intestine (C, D) of nine wild-type and four hPXR mice, using assays specific for the mouse PXR mRNA transcript (A, C) or the human PXR mRNA transcript (B, D);
Figure 17 shows RT-PCR of huPXR transcripts in transgenic mice;
Figure 18 shows Western blotting of PXR protein from wild type and hPXR mice.
Proteins from the livers of treated wild-type and huPXR mice were probed with an antibody specific for the PXR protein. The standard (+ve) was a his-tagged murine PXR;
Figure 19 shows Western blotting of Cyp3a and Cyp2b induction by rifampicin.
Microsomal proteins from the livers of rifampicin treated wild-type and huPXR mice were probed with antibodies for the Cyp 3a11 protein (top panel) and Cyp 2b10 (bottom panel);
Figure 20 shows enzyme activity assays of Cyp3a and Cyp2b10 induction by rifampicin (100mg/kg). Shown are the results from enzyme activity assays on liver microsomes using activity of pentoxyresorufin-O-deethylation (PROD) which is attributed to expression level of Cyp 2b10 and the activity of 7-benzyloxyquinoline (BQ) which is attributed to expression level of Cyp 3a;
Figure 21 shows enzyme activity assays in liver microsomes following induction by rifampicin (3mg/kg and 10mg/kg) - 7-benzyloxyquinoline. Shown are the results from enzyme activity assays on liver microsomes using the activity 7-benzyloxyquinoline;
Figure 22 shows enzyme activity assays in liver microsomes following induction by rifampicin (3mg/kg and 10mg/kg) - 6-beta-hydroxylation of testosterone (p<0.01);
Figure 23 shows enzyme activity assays in liver microsomes following induction by rifampicin (3mg/kg and 10mg/kg) - 16-beta-hydroxylation of testosterone (p<0.05);
Figure 24 shows enzyme activity assays in liver microsomes following induction by TCPOBOB (0.3mg/kg and 1.0mg/kg) - 7-alpha-hydroxylation of testosterone which was constitutively higher in the huPXR animals relative to the wild types;
Figure 25 shows enzyme activity assays in liver microsomes following induction by TCPOBOB (0.3mg/kg and 1.0mg/kg) - 6-beta-hydroxylation of testosterone;
Figure 26 shows enzyme activity assays in liver microsomes following induction by TCPOBOB (0.3mg/kg and 1.0mg/kg) - 16-alpha-hydroxylation of testosterone. These demonstrate that this activity was significantly induced in wild type animals (p<0.05) but not in huPXR animals;
Figure 27 shows enzyme activity assays in liver microsomes following induction by TCPOBOB (0.3mg/kg and 1.0mg/kg) - 16-beta-hydroxylation of testosterone. These demonstrate that this activity was much more marked in wild type than in huPXR animals;
Figure 28 shows PCR confirmation in two double homozygous PXR/CAR humanised mice that the murine PXR gene has been exchanged for the human counterpart.

### Examples

### Example 1: The Humanised PXR/CAR Mouse

The method provides for the humanisation of mice for PXR and CAR alone or as a double humanised form for each of the genes (see Figure 1). We have combined these different humanisations by a step-wise transformation of embryo stem cells (ES cells) rather than by conventional breeding and thus are able to generate PXR/CAR double humanised ES cells, which are usable for subsequent modifications with human DNA sequence encoding a phase-1 or 2 drug-metabolising enzyme human DNA sequences encoding a drug transporter protein.

The application of PhiC31 recombination sites enabled us to knock out both genes by crossing the humanised animal to a Phi31 deleter strain.

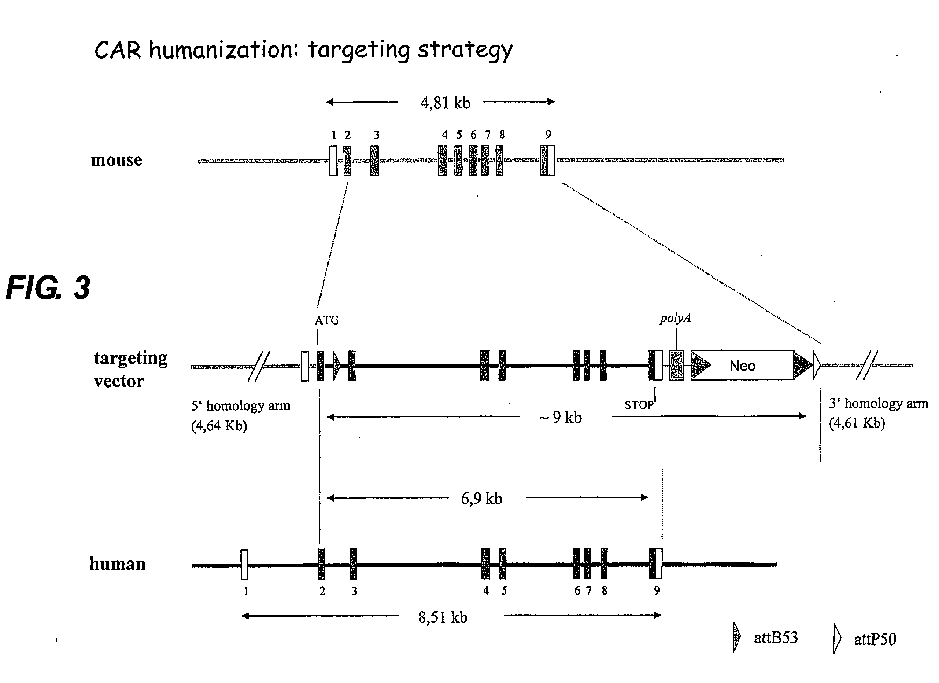

In contrast to existing models, we utilise a "knock-in" approach to replace the endogenous genes by their human orthologues (Figures 2 and 3). Therefore the PXR and CAR genes will be kept under their normal genomic context and expression levels and transcript distribution is advantageously physiologically regulated or controlled. As a result of this, the model of the present invention will resemble the human situation more closely than prior art attempts.

Additionally, genomic structures of human genes are at least partially conserved within the construct of the present invention. In the example of PXR, we have used a composite construct of cDNA and genomic sequences (see Figure 2). Due to the large size of more than 35kb of the human PXR gene, we kept the intron-exon structure solely between 4 and 6, since most splice variants are observed in this genomic region since it is located within the ligand-binding domain. The relatively small size of the human CAR, which comprises roughly 7kb from exon 2-9, enabled us to retain the complete genomic structure in our targeting vector (Figure 3).

The ES cells comprising humanised PXR and/or CAR can be further modified with human genes that regulate drug metabolising enzymes (phase1 and 2) and/or drug transporter proteins. It will be possible to cross the animals cells with humanised PXR/CAR with the HRN mouse below or to create a de novo non-human transgenic animal with all of the aforementioned criteria.

### Example 2: The HRN™ Mouse

All P450s require reducing electrons supplied by the enzyme cytochrome P450 reductase (CPR). Deletion of CPR therefore simultaneously inactivates all P450s. While CPR deletion is lethal in the embryo, HRN™ mice use a developmentally regulated conditional CPR deletion targeted to post-natal liver cells. HRN™ mice therefore survive to adulthood and can breed while nevertheless completely lacking hepatic P450-mediated metabolism (Henderson CJ et al. J Biol Chem. 278:13480-6, 2003). They therefore provide a suitable background on which to express human P450 activities in order to achieve P450 humanisation.

### Transgenic Mouse Production

An adenoviral vector may be used to introduce the human P450/CPR combination to HRN™ cells. Alternatively, germ line transgenic animals incorporating the same transgenes can be produced. This is achieved by first generating transgenic mice incorporating the selected CYP3A4/CPR humanisation transgenes and then crossbreeding these with HRN™ mice to produce CYP3A4-humanised animals. Production of CYP3A4/CPR transgenic mice is achieved by using targeted transfection of embryonic stem cells and subsequent blastocyst injection. Crossbreeding of CYP3A4/CPR transgenics with HRN™ to produce P450-humanised animals may be used for the production of multi-P450-humanised mice. Alternatively, embryonic stem cells may be produced where the CPR gene is flanked by *lox*P sites and where expression sequences for targeted human P450(s) and CPR or for human P450-CPR fusion protein(s) have been introduced. Animals derived from such embryonic stem cells may then be crossbred with various animal strains in which cre recombinase is expressed under the control of different promoters to produce offspring P450-humanised in different tissues or under different induction conditions, depending on the tissue specificity or inducibility of the promoter controlling cre recombinase expression.

### Humanisation Strategies

In order to establish the optimal method of expressing functional human P450 activities in HRN™ cells, experimental transgenes encoding cytosolic fusion proteins, targeted fusion proteins, targeted CPR with separate cytosolic P450s are compared and evaluated. In each case, the ability of the P450 to interact with the CPR component is determined by expressing these alternatives in appropriate cell culture systems and then testing them *in vivo* by adenovirus transfection of HRN™ mice.

### Examples 3: Humanization projects

### Type 1: Expression of human cDNA from the corresponding mouse promoter

### Projects: MDR1-humanization

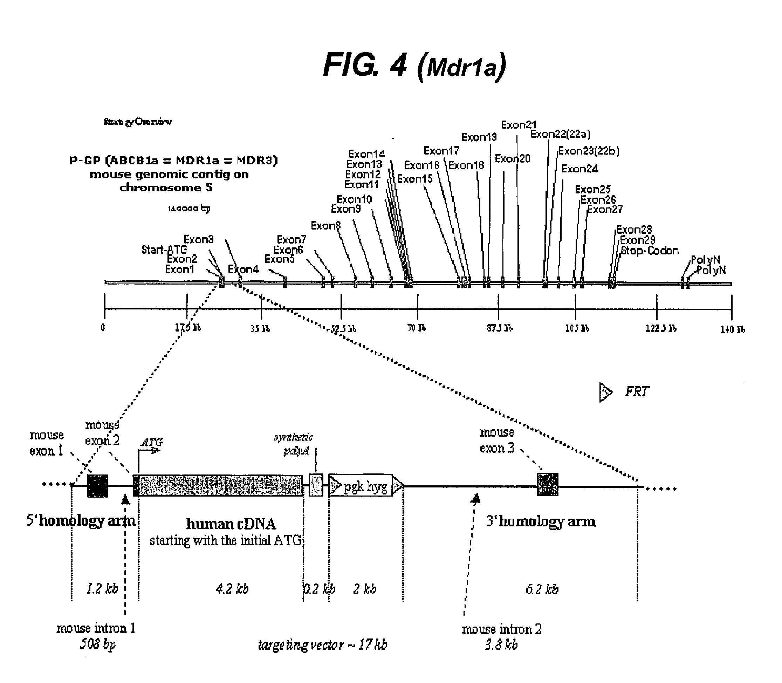

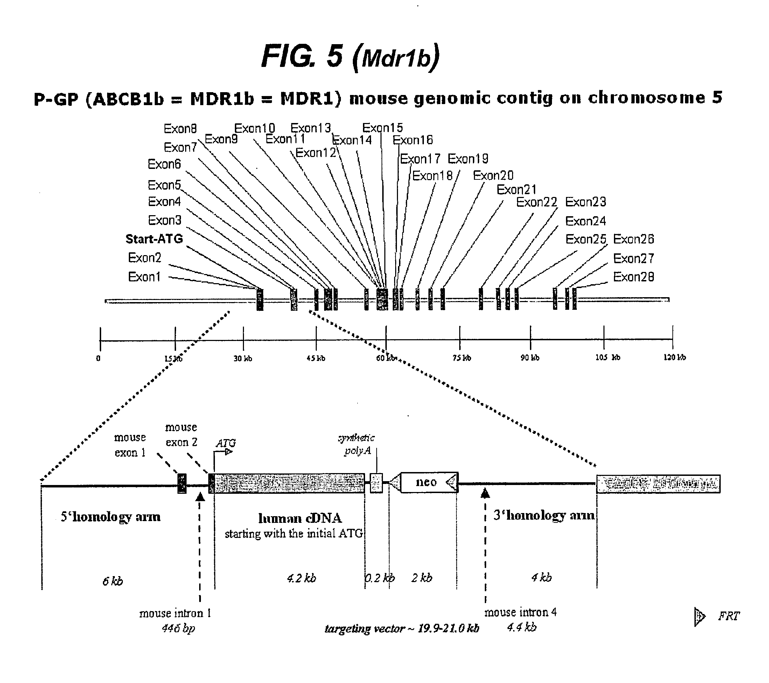

**Methods:** The targeting vectors are constructed with standard molecular cloning procedures. These vectors are designed in such a way, that the cDNA of human MDR1 is fused to the translational start site of the corresponding mouse genes (*Mdr1a* and *Mdr1b*). The Mdrl a targeting vector carries an FRT-flanked hygromycin resistance cassette, the Mdrlb targeting vector an F3-flanked neomycin resistance cassette. In both cases the transcripts are terminated by a polyA motif. In case of *Mdr1a* the targeting event removes the 3'part of exon2, in case of Mdr1b the 3'part of exon2 to exon4 are deleted. See Figures 4 and 5.

In two consecutive rounds of standard electroporation in C57BL/6N mouse ES cells both mouse genes are modified due to homologous recombination. Clones from each round of transfection are selected with G418 and hygromycin, respectively, and positive clones are identified by Southerm blot analysis. Selected clones are expanded, injected into *BALBc*-blastocysts and transferred into foster mothers according to standard operation procedures. Litters from these fosters are visually inspected and chimerism is determined by hair colour. Highly chimeric animals are used for further breeding in a C57BL/6N genetic background. Selection markers are removed *in vivo* by crossing to an FLP-deleter strain.

### Type 2: Expression of a fusion of mouse gene and human cDNA from the corresponding mouse promoter

### Projects: MRP2-humanization

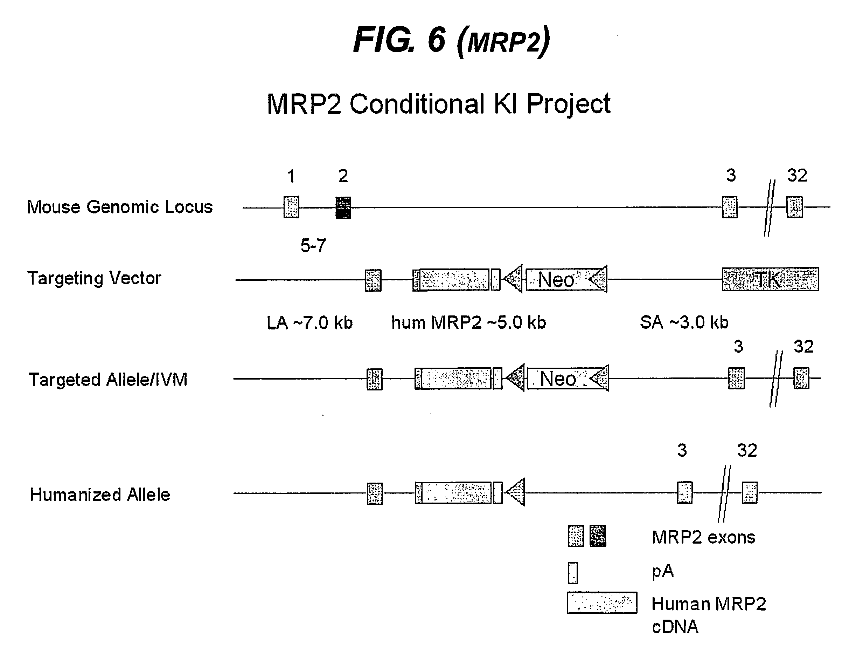

**Methods:** The targeting vector is constructed with standard molecular cloning procedures. The vector is designed in such a way, that the "Leader sequence" of the mouse protein, which is encoded by exon1, will be retained. The human cDNA without sequences from exon1 is introduced into exon2. The original splice sites for mouse intron1 will be retained, so that this construct potentially encodes a fusion protein of amino acids from mouse exon1 and human exon2-32. The transcript is terminated by a polyA motif. The targeting vector carries an FRT-flanked neomycin resistance cassette (see Figure 6).

The targeting vector is transfected by standard electroporation into C57BL/6N mouse ES cells. Clones are selected with G418 and positive clones are identified by Southerm blot analysis. Selected clones are expanded, injected into *BALBc-*blastocysts and transferred into foster mothers according to standard operation procedures. Litters from these fosters are visually inspected and chimerism is determined by hair colour. Highly chimeric animals are used for further breeding in a C57BL/6N genetic background. Selection markers are removed *in vivo* by crossing to an FLP-deleter strain.

### Type 3: Expression of a hybrid of human cDNA and genomic sequences from the corresponding mouse promoter

### Projects: PXR humanization

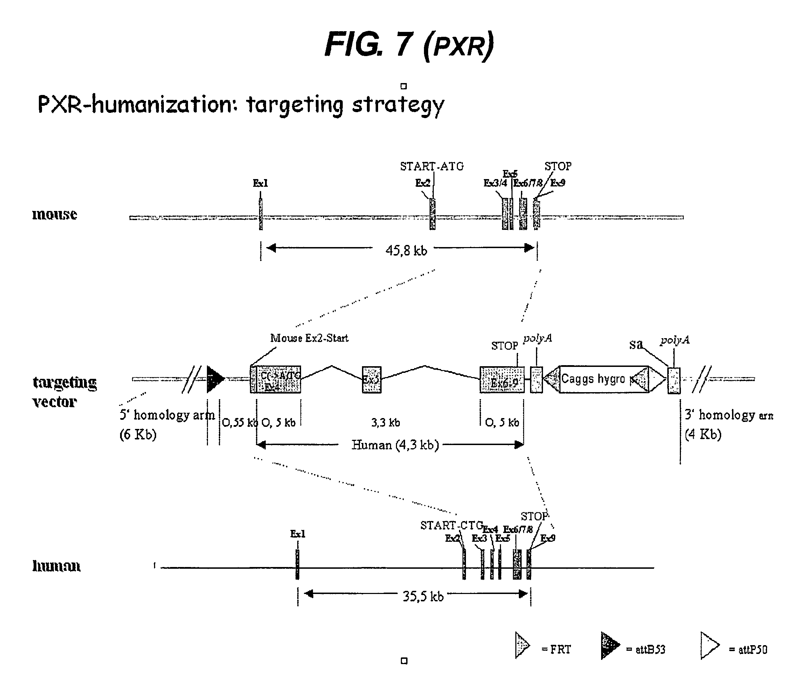

**Methods:** The targeting vector is constructed with standard molecular cloning procedures. The vector is designed in such a way, that a hybrid of human PXR cDNA and genomic sequences is fused to the translational start site of the mouse PXR gene, whereby the mouse Start-ATG is retained. The human PXR sequence contains the cDNA of exon1-4, genomic sequences of intron4, exon5 and intron5 and cDNA of exon6-9. The transcript is terminated by a polyA motif. The targeting vector carries an FRT-flanked hygromycin resistance cassette and a splice acceptor polyA motif 3' to the selection cassette. Furthermore, att sites have been inserted into mouse intron1 and 3' to the splice acceptor polyA motif, which allow the generation of a PXR knock out by removal of the intermediate sequences with the site-specific Phi-C31 recombinase (see Figure 7).

The targeting vector is transfected by standard electroporation into C57BL/6N mouse ES cells. Clones are selected with hygromycin and positive clones are identified by Southerm blot analysis. Selected clones are expanded, injected into *BALBc-*blastocysts and transferred into foster mothers according to standard operation procedures. Litters from these fosters are visually inspected and chimerism is determined by hair colour. Highly chimeric animals are used for further breeding in a C57BL/6N genetic background. Selection markers are removed *in vivo* by crossing to an FLP-deleter strain.

### Confirmation that the murine PXR gene has been exchanged for the human counterpart

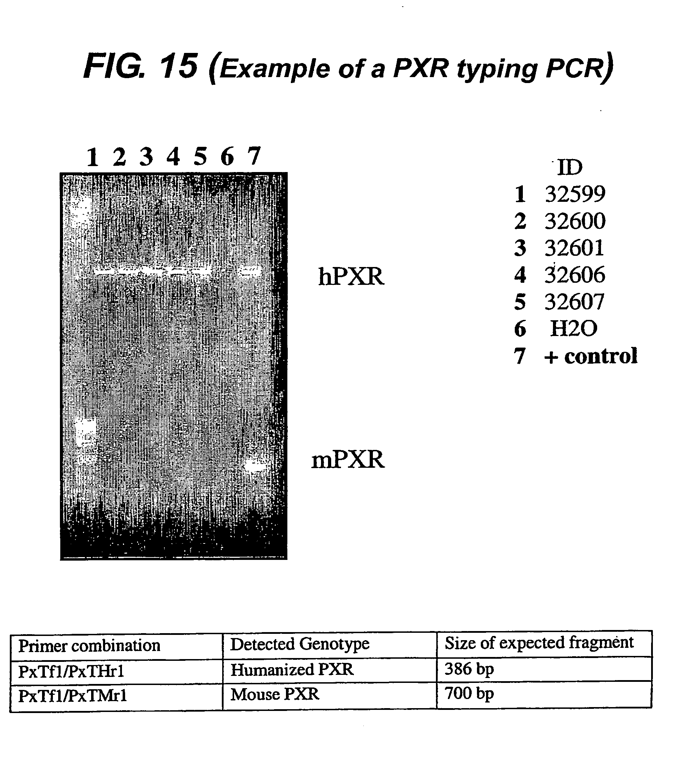

Humanised mice for PXR have been generated using the above strategy and are phenotypically normal following visual inspection. They have been typed using PCR (see Figure 15). The mice live to at least 3 months age. Examples include male "30643": DOB 12.12.2004. Remained alive at 19.04.05. Female "30792": DOB 19.12.04. Remained alive at 19.04.05. These mice can be successfully bred:
□ 30643 x 30792 set up at 01.02.2004
□ Litter born 22.02.2005 - 6 pups - weaned 30.03.2005
□ Litter born at 06.04.2005 - 7 pups - to be weaned

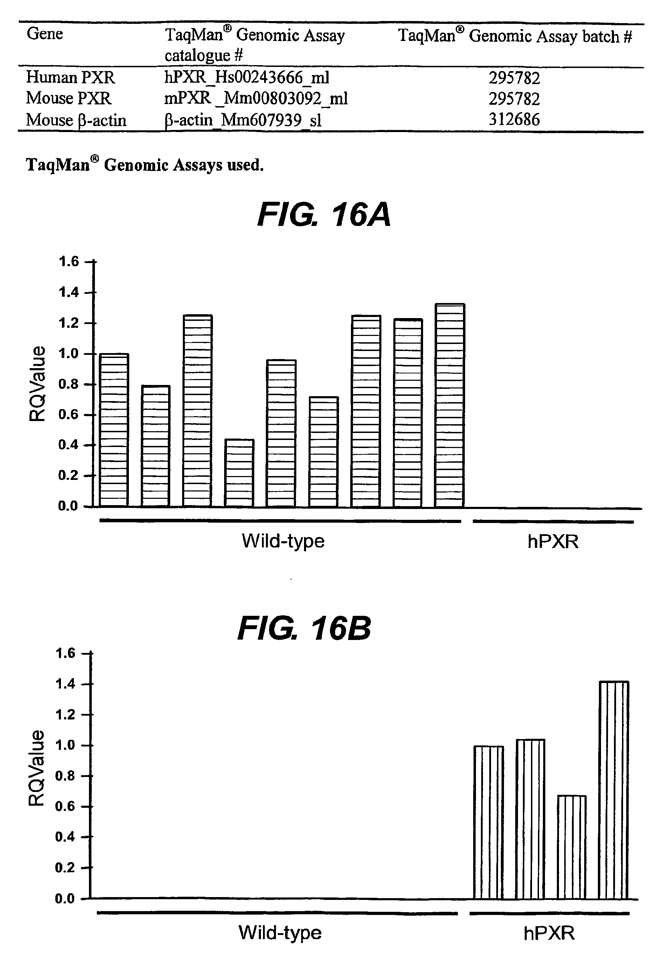

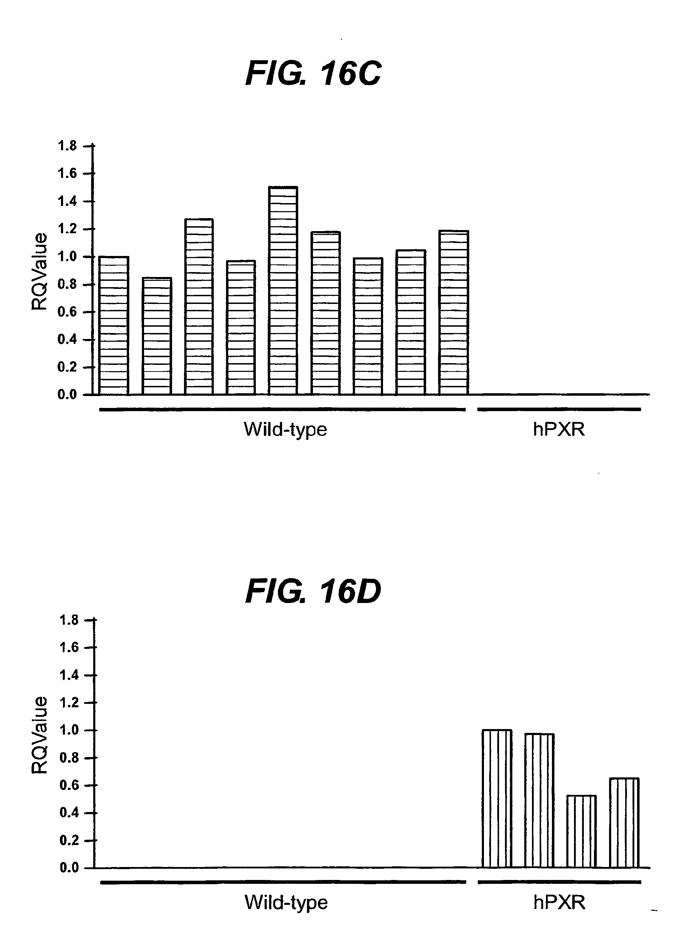

Further typing has been performed using Taqman^{®} analysis (see Figure 16). This analysis clearly demonstrated the presence of a murine PXR transcript only in the wild type animals and the presence of the human transcript only in the transgenic mouse lines demonstrated that gene exchange had taken place and that the gene construct was being actively transcribed in the correct tissues.

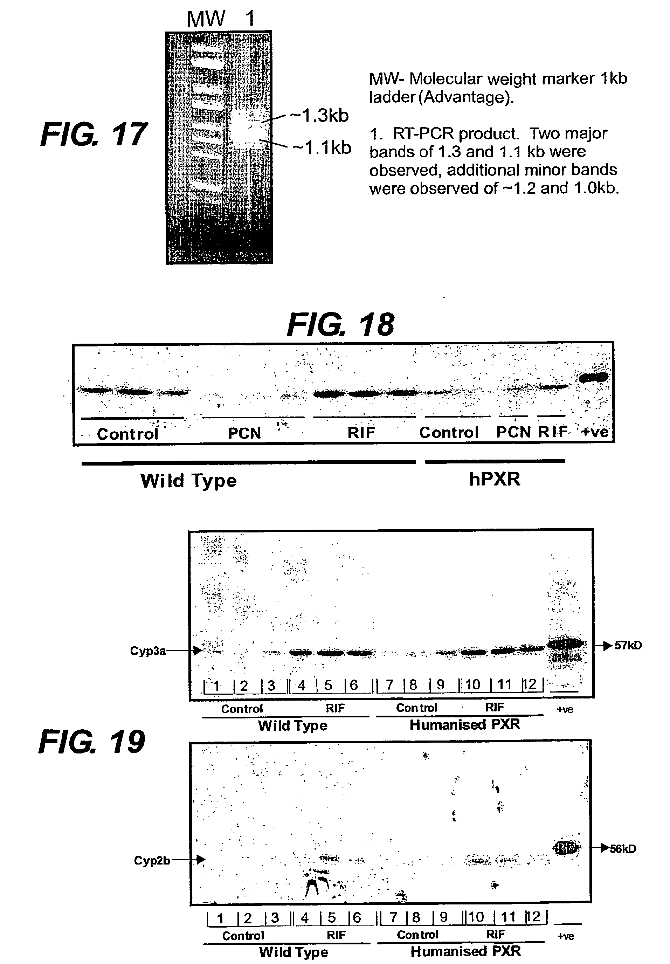

RT-PCR was then performed to confirm the presence of a full-length human PXR transcript in the humanised mice (see Figure 17). This analysis demonstrated the presence of two transcripts of size 1.3 and 1.1 kilo-bases, indicating that the entire humanised PXR gene had been transcribed. The size of the fragments obtained indicated that the correct, as well as alternative spliced variants were present.

Sequence analysis of the full length transcript confirmed that an open reading frame for the entire PXR mRNA had been produced. The presence of PXR was subsequently confirmed by Western blot analysis using an antibody raised against the murine PXR.

### Demonstration that the human PXR was functional

In order to demonstrate that the human PXR protein was functional, mice were challenged with PXR-activating compounds including pregnenolone-16α carbonitrile (PCN) and rifampicin (Rif).

Dosing solutions were prepared on the day of administration by adding corn oil to the requisite quantity of test substance and stirring to obtain a solution or fine suspension. The concentration of PCN was 10mg/ml and Rif 2.5mg/ml of supplied chemical, without any correction for purity. Records of preparation were retained.

Control animals were administered vehicle (corn oil) daily by intraperitoneal injection. The volume of vehicle and solutions of inducing agents administered was 10ml/kg bodyweight. This route of administration was chosen for consistency with previously published work.

Animals received 4 daily doses and were killed approximately 24 hours after the last dose.

Quantification of Cyp3a11, Cyp2b10 and PXR protein in liver microsomes of all mice samples was carried out by SDS-PAGE and Western blotting. To detect murine and human PXR protein, an antibody (RF8) was employed. For the identification of Cyp3a and Cyp2b, anti-cyp2b antibodies were used, (CH32 and CH4, respectively). Results are shown in Figure 18.

These data demonstrated a protein of the correct molecular weight identified in both the wild type mice and the humanised PXR mice. The molecular weights of the two proteins were shown to be the same, which is consistent with the calculated molecular weight from the predicted amino acid sequence. In addition, it was interesting to note that the level of expression of PXR between the murine and humanised animals was very similar, consistent with the fact that expression is driven off the same promoter.

Analysis for the PXR inducible proteins in the cytochrome P450 Cyp3a gene family demonstrated a marked induction in the humanised PXR mouse (see Figure 19). Interestingly, the cytochrome P450 Cyp2b10 was not markedly induced by this treatment, demonstrating that the induction was via the PXR protein and not by the alternative transcription factor, constitutive androstane receptor (CAR). In this experiment, Cyp3a11 was also induced by rifampicin in wild type animals. Although rifampicin is reported to exhibit species differences in its ability to interact with PXR, the fact that no differences were observed here could be accounted for by the high dose of compound used.

### Enzyme activity assays

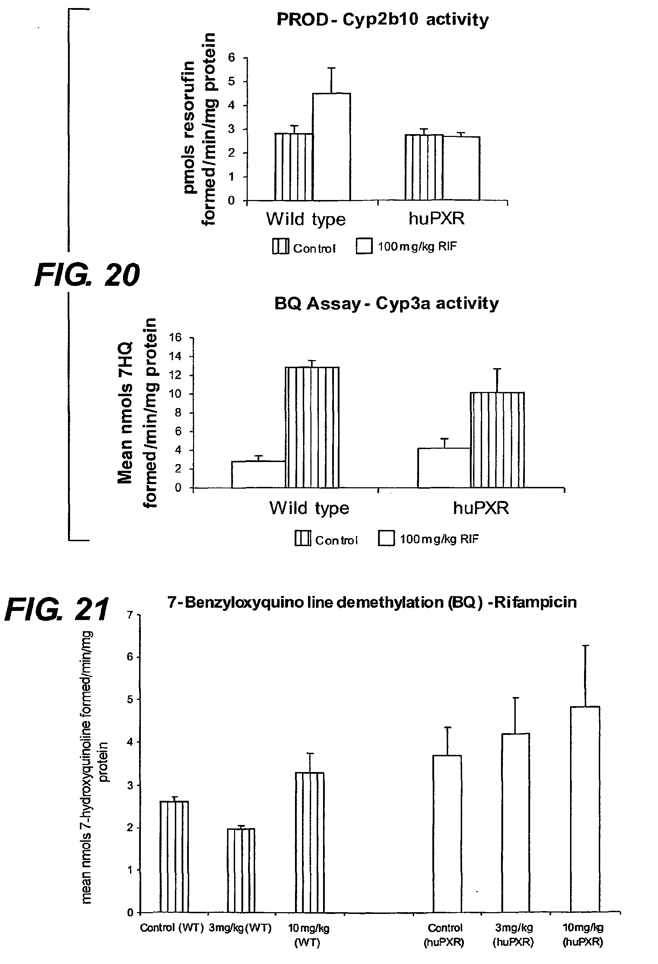

Using enzyme activity assays on liver microsomes, we were able to confirm the Western blot results. The activity in pentoxyresorufin-O-deethylation (PROD) is attributed to expression level of Cyp 2b10 and the activity of 7-benzyloxyquinoline (BQ) is attributed to expression level of Cyp 3a. The enzyme activity data for Cyp3a and Cyp2b induction by rifampicin are shown in Figure 20.

### Induction study in huPXR mice using low doses of rifampicin and TCPOBOP

In order to demonstrate strain differences between wild type and the huPXR mouse, mice were challenged with low doses of rifampicin or TCPOBOP. These compounds were chosen because rifampicin has been reported to be a more efficient inducer of human PXR than murine transcription factor, and TCPOBOP is reported to be a more efficient inducer of cytochrome P450 gene expression in the mouse than in human systems.

The study consisted of 3 animals per group. Control groups consisted of mice treated with either corn oil daily for 4 days or mice administered a single injection of corn oil. The treated mice were dosed with inducing agents as detailed in Table 3.1.

### Table 3.1: - Experimental design for the induction study in huPXR mice using low doses of rifampicin and TCPOBOP

| Mouse Strain | Inducing agent | Dose (mg/kg) | Vehicle | Regimen |
|---|---|---|---|---|
| C57BL/6J | Control I | - | Corn oil | 4 x ip daily |
| huPXR | Control I | - | Corn oil | 4 x ip daily |
| C57BL/6J | RIF | 3 | Corn oil | 4 x ip daily |
| C57BL/6J | RIF | 10 | Corn oil | 4 x ip daily |
| huPXR | RIF | 3 | Corn oil | 4 x ip daily |
| huPXR | RIF | 10 | Corn oil | 4 x ip daily |
| C57BL/6J | Control II | - | Corn oil | Single ip |
| huPXR | Control II | - | Corn oil | Single ip |
| C57BL/6J | TCPOBOP | 0.3 | Corn oil | Single ip |
| C57BL/6J | TCPOBOP | 1 | Corn oil | Single ip |
| huPXR | TCPOBOP | 0.3 | Corn oil | Single ip |
| huPXR | TCPOBOP | 1 | Corn oil | Single ip |

PXR activity was determined by measuring the metabolism of a number of cytochrome P450 substrates which are metabolised to different extents by different cytochrome P450 enzymes. The following assays were performed:
- 7-benzyloxyquinoline (BQ) activity was determined in liver microsomes.
- Testosterone hydroxylase activity was determined in liver microsomes (LMS HPLC-006).
- The specific activities of these reactions was expressed per mg protein as determined using the Lowry assay (LMS Spec-0001).

In the first experiment, 7-benzyloxyquinoline was taken as substrate. In the rifampicin treated animals, in the first instance, there was clearly a difference between the background CYP3A enzyme activity between wild type and humanised PXR mice (Figure 21). In addition, although this enzyme activity was not inducible by rifampicin, there was a marked difference in enzyme activity between the wild type and humanised PXR animals at a dose of 3 mg/kg.

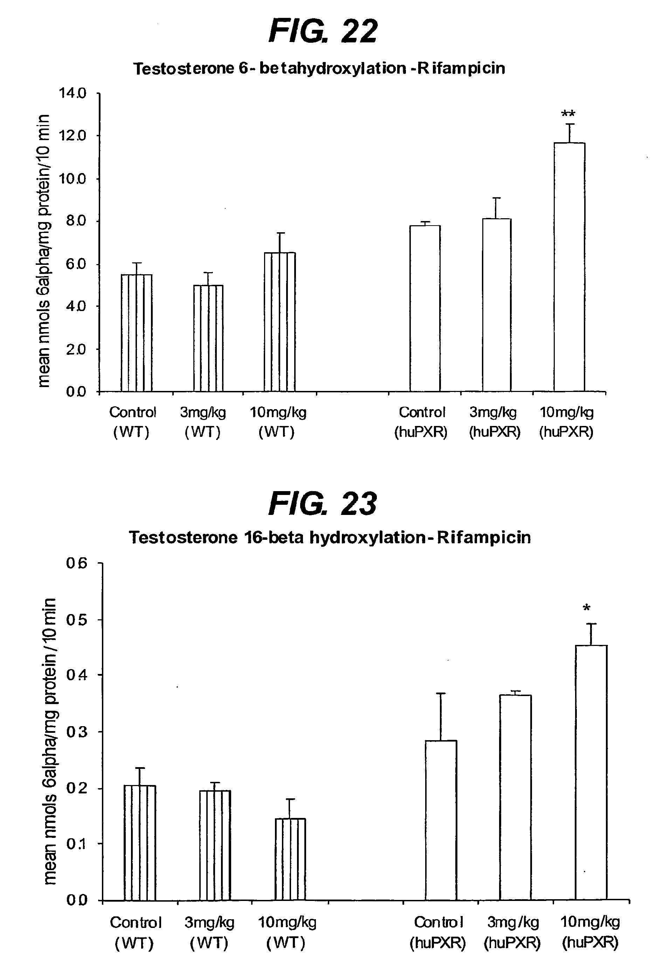

In subsequent experiments, testosterone hydroxylation was measured in liver microsome fractions from wild type and huPXR mice. It was interesting to note that at a dose of 10 mg/kg there was no induction of 6-beta- hydroxylation of testosterone in wild type animals whereas there was a significant induction in the human PXR animals, demonstrating an altered sensitivity of these mice to rifampicin relative to the wild type (Figure 22).

There was a similar finding for the measurement of the 16-beta-hydroxylation of testosterone which, similar to the 7-benzyloxyquinoline demethylation, exhibited higher activity in controls (Figure 23). Again, this activity was induced in the huPXR animals but not in the wild type. Indeed, the activity was approximately 3-fold in the huPXR mice.

There was a similar finding in the 2-alpha-hydroxylation of testosterone. In addition, the induction of Cyp3a11 by rifampicin demonstrates that the huPXR is functionally active in the huPXR mice.

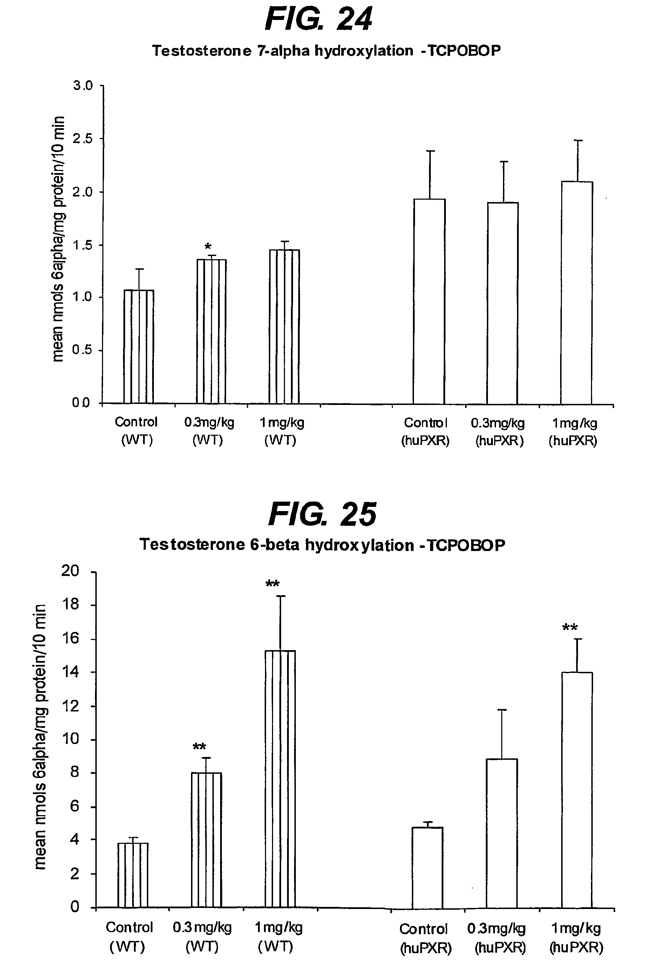

In experiments using the inducing agent TCPOBOP the hepatic microsomal metabolism of testosterone was also measured. Again clear differences between the wild type and the huPXR animals were observed. In particular, the 7-alpha-hydroxylation of testosterone was constitutively higher in the huPXR animals relative to the wild types (Figure 24).

The 6-beta-hydroxylation of testosterone was inducible in both strains of mouse. Although there were no significant differences between the wild type and huPXR mice, this does demonstrate again that the human PXR is active (Figure 25).

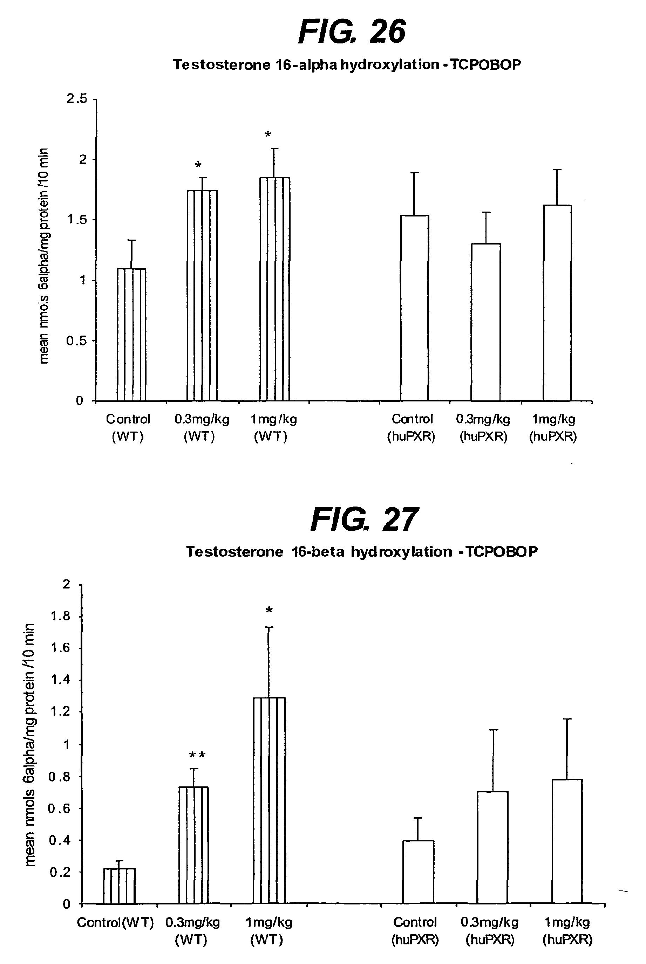

Consistent with the strain differences in wild type and human PXR, there were marked differences in the sensitivity of the mouse lines to induction by TCPOBOP. In the case of testosterone 16-alpha-hydroxylation, this activity was significantly induced in wild type animals but not in huPXR animals, and of particular interest was the observation that the induction of testosterone 16-beta-hydroxylation was much more marked in wild type than in huPXR animals (see Figures 26 and 27 respectively). Indeed, at a dose of 1mg/kg, induction of testosterone 16-beta-hydroxylation was approximately 6-fold in wild type animals but only 1.7-fold in the huPXR animals. This again demonstrates a reduced sensitivity of the humanised mice relative to controls.

This is the first report of a mouse model where the human PXR gene has been exchanged for the endogenous murine protein. The advantages of such a model relative to other PXR humanised mice lie in the fact that:-
- The construct used allows the studies to be undertaken to understand the role of alternative splicing of PXR in controlling its level of expression.
- Use of the endogenous mouse promoter PXR is expressed in all tissues in a similar fashion to those of the endogenous gene.
- The level of PXR expression seems to be very similar to the endogenous gene, therefore problems with potential over/under expression are reduced.
- The currently available humanised PXR models use the albumin promoter to drive human PXR which has been crossed into a PXR null background. Therefore, PXR is expressed at very high levels in this model and there is no PXR in any tissue other than the liver. This severely compromises the use of this model to understand the role of hPXR in controlling gene expression in the GI tract or at the blood brain barrier or, indeed, in any other tissue.

Humanised PXR mice have a number of utilities. For example, such mice can be used to understand the role of human PXR in the control of gene expression in any tissue in the mouse. They can also be used to evaluate whether drugs in development or environmental chemicals have the capacity to modulate human PXR functions in a manner which may be beneficial or deleterious. The model also allows studies into understanding the role of human PXR in mediating the potential toxic effects of drugs or chemicals which may result from perturbations in, for example, bile acid homeostasis and therefore their relevance to man. The model can also be used to evaluate chemicals that may be either antagonistic or agonistic to this signalling pathway.

### Type 4: Expression of a human genomic sequence from the corresponding mouse promoter

### Projects: CAR humanization

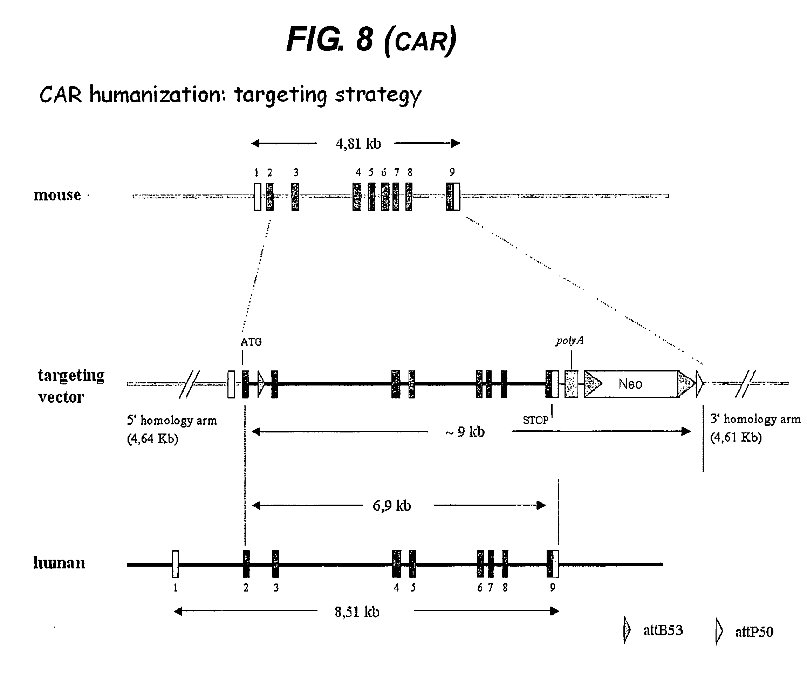

Methods: The targeting vector is constructed with standard molecular cloning procedures. The vector is designed in such a way, that the genomic human CAR sequence is fused to the translational start site of the mouse CAR gene. The human CAR sequence contains all genomic sequences of exons 1-9, except the 5' and 3'UTRs, which are retained from the mouse genome. All other parts of the coding sequences of the mouse CAR gene will be deleted. The transcript is terminated by a polyA motif. The targeting vector carries an FRT-flanked neomycin resistance cassette. Furthermore, att sites have been inserted into human intron2 and 3' to the selection marker, which allow the generation of a CAR knock out by removal of the intermediate sequences with the site-specific Phi-C31 recombinase (see Figure 8).

The targeting vector is transfected by standard electroporation into PXR humanized C57BL/6N mouse ES cells. Clones are selected with hygromycin and positive clones are identified by Southern blot analysis. Selected clones are expanded, injected into BALBc-blastocysts and transferred into foster mothers according to standard operating procedures. Litters from these fosters are visually inspected and chimerism is determined by hair colour. Highly chimeric animals are used for further breeding in a C57BL/6N genetic background. Selection markers are removed *in vivo* by crossing to an FLP-deleter strain.

The targeting vector is transfected by standard electroporation into C57BL/6N mouse ES cells. Clones are selected with hygromycin and positive clones are identified by Southerm blot analysis. Selected clones are expanded, injected into *BALBc-*blastocysts and transferred into foster mothers according to standard operation procedures. Litters from these fosters are visually inspected and chimerism is determined by hair colour. Highly chimeric animals are used for further breeding in a C57BL/6N genetic background. Selection markers are removed *in vivo* by crossing to an FLP-deleter strain.

### PCR confirmation in two double homozygous PXR/CAR humanised mice that the murine PXR gene has been exchanged for the human counterpart

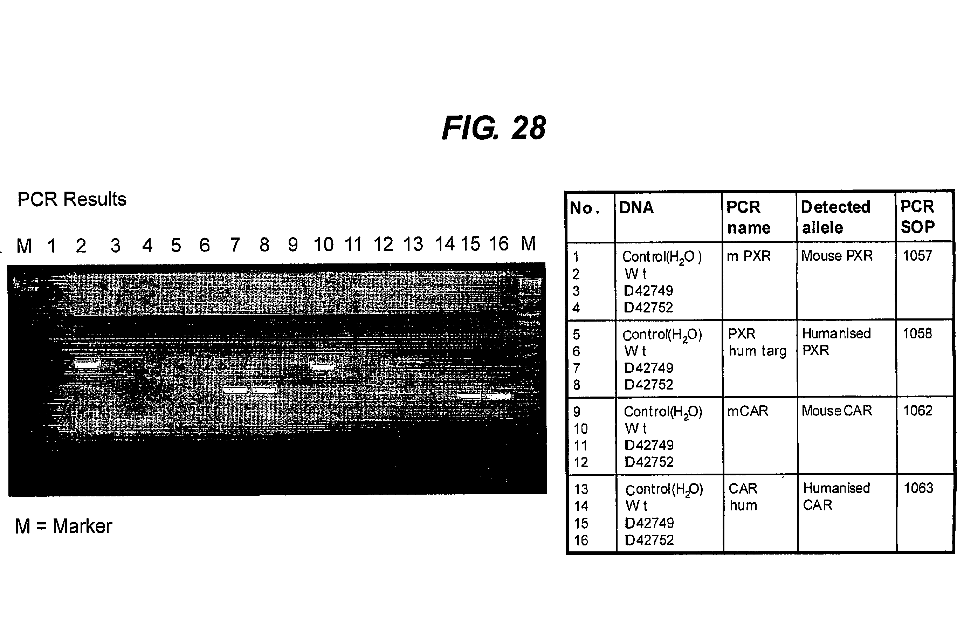

Humanised mice for PXR and CAR were generated using mice which contained humanised PXR and crossing these into mice which contained humanised CAR to produce mice containing both humanised PXR and humanised CAR. The mice are phenotypically normal following visual inspection. They have been typed using PCR (see Figure 28) and are homozygously humanised for PXR and CAR. Examples include mice designated "42749" and "42752".

### Pentobarbitone sleepin test

In this experiment, the activity of these transcription factors in combination was determined by measuring the barbiturate induced sleeping time. Sleeping time has been known for many years to be directly proportional to the hepatic cytochrome P450 activity and this activity can be at least in part ascribed to the P450 levels in the liver determined by CAR and PXR function.

Mice were given a single intraperitoneal dose of Narcoren (sodium pentobarbitone; purchased via a Veterinary Consultant; distributed by Merial GmbH, Germany) at 25mg/kg of body weight. The time taken for the mice to lose, and subsequently to regain, their righting reflex was measured. Results are given in Table 4.1 below:

### Table 4.1; results of pentobarbitone sleeping test

| Genotype | Sex | Age (weeks) | ID | Weight (g) | Sleeping time (min) |
|---|---|---|---|---|---|
| wt | male | 10 | 42912 | 21.2 | 21 |
| PXR/CAR hum | male | 10 | 42749 | 24.8 | 34 |

Whereas wild type mice given a narcotic dose of pentobarbitone slept for 21 minutes, the double humanised mice for CAR and PXR slept for 34 minutes. These mice therefore demonstrate a significant difference to their wild type controls indicating that the double humanised mouse has a marked difference in its response to drugs relative to the wild type animals.

### Summary of work on Types 3 and 4 above

A model has been developed where human PXR is expressed in both the liver and GI tract of mice in the predicted fashion at levels equivalent to those of the endogenous gene. The PXR protein has been shown to be functional as the mice are responsive to compounds known to induce gene expression via this pathway. A strain difference between wild type and the humanised mice was demonstrated and the humanised mice were shown to be more responsive to a compound known to be more active to hPXR. In addition to the animal experiments carried out with the humanised PXR mice, mice which contained humanised PXR have also been crossed into mice which contained humanised CAR to produce mice containing both humanised PXR and humanised CAR.

### Type 5: Expression of a hybrid of human cDNA and genomic sequences from the corresponding human promoter by insertion into the ROSA26 locus

### Projects: CYP3A4 and CYP2C9 humanization

**Methods:** The targeting vectors are constructed with standard molecular cloning procedures. The basic ROSA26 targeting vector is designed in a way, that the neomycin gene will be expressed from the endogenous ROSA26 promoter in correctly targeted ES clones. The Neo transcript is terminated by a polyA motif.

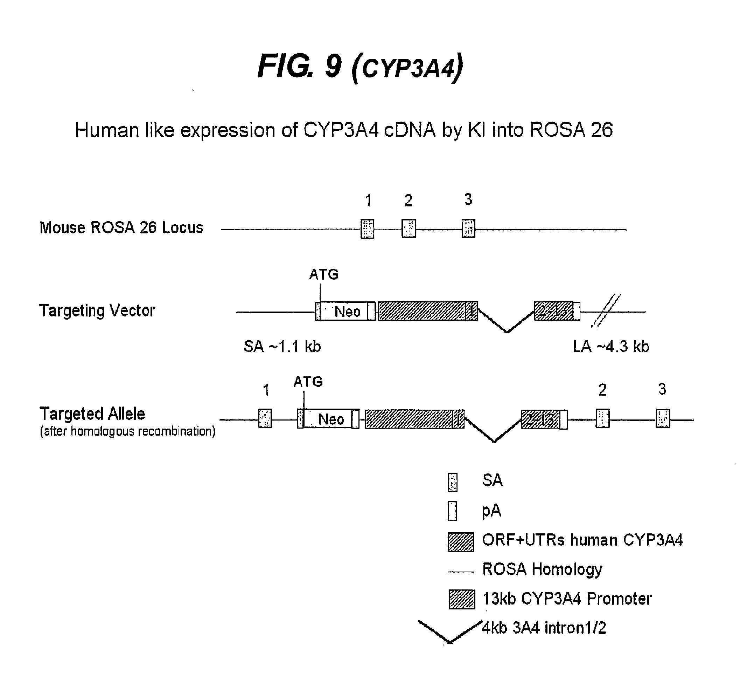

In case of CYP3A4 the humanization cassette, 3' to the selection marker, contains the 13kb human CYP3A4 promoter, exon1 and intron1 as in the normal genomic constitution and a human cDNA consisting of exons2-13. The transcript is terminated by a polyA motif (see Figure 9).

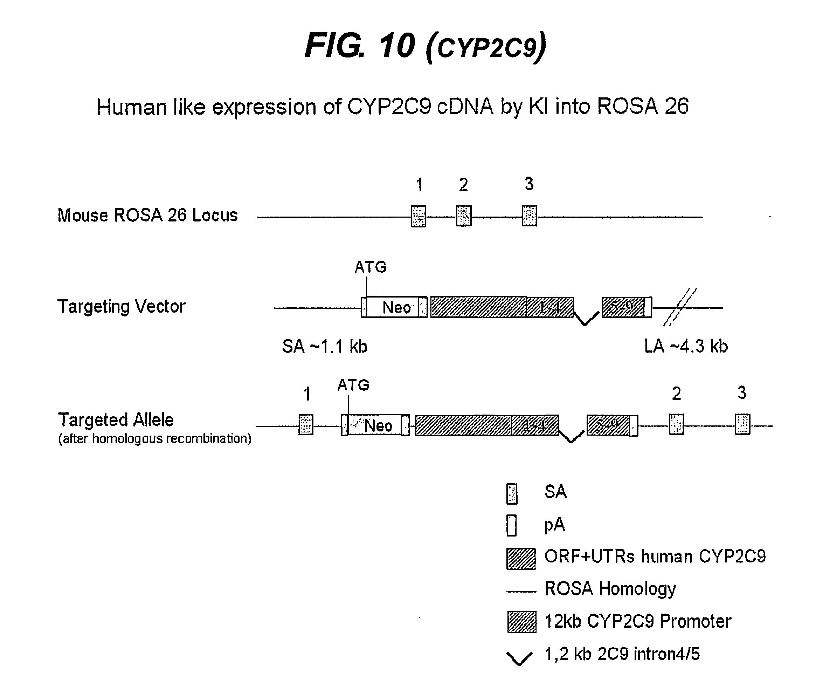

In case of CYP2C9 the humanization cassette, 3' to the selection marker, contains the 12kb human CYP2C9 promoter, a human cDNA of exons1-4, intron4 and a cDNA of exons5-9. The transcript is terminated by a polyA motif (see Figure 10).

The targeting vector is transfected by standard electroporation into C57BL/6N mouse ES cells. Clones are selected with G418 and positive clones are identified by Southern blot analysis. Selected clones are expanded, injected into *BALBc-*blastocysts and transferred into foster mothers according to standard operation procedures. Litters from these fosters are visually inspected and chimerism is determined by hair colour. Highly chimeric animals are used for further breeding in a C57BL/6N genetic background.

### Type 6: Replacement of large genomic regions (Cluster exchanges)

### Projects: CYP3A-cluster exchange, CYP2C-cluster exchange and UGT-humanization

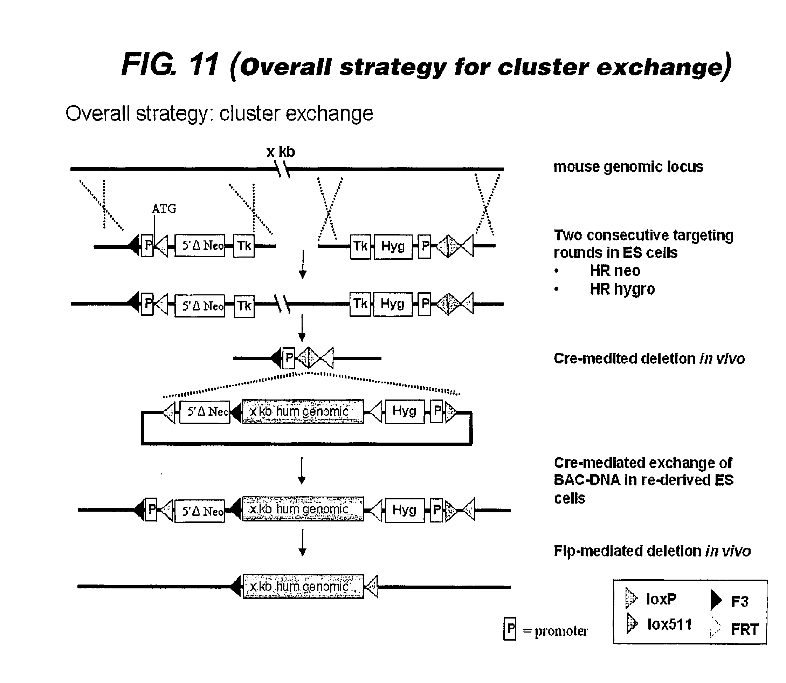

**Methods:** The targeting vectors are constructed with standard molecular cloning procedures. The general principle is that two kinds of targeting vectors are constructed and used for two consecutive rounds of transfection by standard electroporation into C57BL/6N mouse ES cells. The first vector contains a functional TK cassette and a 5' deleted Neo gene interrupted from its tranlational Start-ATG and promoter by a wt-loxP site. The second vector carries functional TK and Hygromycin cassettes, a wt-loxP and a lox511 site. The final targeting vectors for each of the cluster exchanges are designed in such a way, that in correctly targeted ES clones the genomic sequences intermediate to the wt-loxP sites can be removed by Cre-mediated deletion. This results in a knock out of the loxP-flanked gene cluster. Modified Bacterial Artificial Chromosomes (BACs) will be used to introduce human sequences by Cre-mediated insertion into re-derived ES cells. The selection cassettes flanking the humanized clusters can be removed by FLP mediated deletion (see Figure 11).

### Example 4: Reporter projects

### Projects: CYP3A4-, CYP2C9-, CYP2C19-, CYP2B6-, CYP2D6- and MDR1-reporter

**Methods:** The targeting vectors are constructed with standard molecular cloning procedures. For all reporters human BACs are modified in such a way, that a reporter gene is fused to the translational start site of the corresponding human gene. Modified BACs carry an FRT-flanked Keo cassette, permitting the selection of bacterial colonies with kanamycin and mouse ES cell clones with G418. In case of CYP3A4, CYP2C9 and CYP2C19 the transcript of the reporter gene is not terminated by a polyA motif, but the constructs are designed such, that the endogenous polyA motif is potentially used. These constructs are therefore dependent on a correct splicing of the exons 3' to the reporter (see Figure 12).

In case of CYP2D6 and CYP2B6 the transcript of the reporter gene is terminated by a polyA motif linked to the reporter gene with a synthetic intron (see Figure 13).

In case of MDR1 the transcript of the reporter gene is terminated by a polyA motif without an additional intron (see Figure 14).

The modified BAC are linearized with NotI and transfected into C57BL/6N mouse ES cells either by standard electroporation or lipofection with lipofectamin2000. Clones are selected with G418 and positive clones with randomly integrated DNA are identified by PCR analysis. Selected clones are expanded, injected into *BALBc-*blastocysts and transferred into foster mothers according to standard operation procedures. Litters from these fosters are visually inspected and chimerism is determined by hair colour. Highly chimeric animals are used for further breeding in a C57BL/6N genetic background. Selection markers are removed *in vivo* by crossing to an FLP-deleter strain.

## Claims

1. A transgenic mouse, tissue or cells derived therefrom incorporating in its genome at least one human DNA sequence encoding at least one transcription factor under the control of a transcription factor promoter and whose endogenous equivalent genes have been annulled, the mouse, tissue or cells derived therefrom further incorporating at least one or more of the following further human DNA sequences selected from the group comprising:
(i) a DNA sequence encoding a phase-1 drug-metabolising enzyme;
(ii) a DNA sequence encoding a phase-2 drug-metabolising enzyme; and/or
(iii) a DNA sequence encoding a drug transporter protein
and whose endogenous equivalent gene or genes have been annulled;
wherein the human sequence encoding the at least one transcription factor is inserted at the point in the genome where the endogenous equivalent gene naturally occurs; and
wherein the transcription factor is selected from the group comprising the pregnane X receptor (PXR/SXR) and the constitutive androstane receptor (CAR) or a combination thereof.

2. A transgenic mouse, tissue or cells derived therefrom according to claim 1 wherein the human DNA encoding a transcription factor comprises both the pregnane X receptor (PXR) and the constitutive androstane receptor (CAR).

3. A transgenic mouse, tissue or cells derived therefrom according to any preceding claim wherein the human DNA encoding a phase-1 drug-metabolising enzyme is selected from the group comprising the cytochromes P450.

4. A transgenic mouse, tissue or cells derived therefrom according to claim 3 wherein the P450 comprises any one or more of the following isoforms CYP3A4, CYP2C9, CYP2C 19, CYP2D6, CYP1A2, CYP2C8 and CYP2B6.

5. A transgenic mouse, tissue or cells derived therefrom according to claim 3 or claim 4 wherein the human DNA encoding a phase-1 drug-metabolising enzyme comprises the CYP3A and/or the CYP2C gene clusters.

6. A transgenic mouse, tissue or cells derived therefrom according to any preceding claim wherein the human DNA encoding a phase-2 drug-metabolising enzyme comprises any one or more of the transferases selected from the group comprising a glucuronyl transferase, glutathione transferase, sulphonyl transferase and acetyl transferase.

7. A transgenic mouse, tissue or cells derived therefrom according to claim 6 wherein the human DNA encoding a phase-2 drug-metabolising enzyme comprises the UGT1A gene cluster.

8. A transgenic mouse, tissue or cells derived therefrom according to any preceding claim wherein the human DNA encoding a drug transporter protein is selected from the group comprising an ATP-binding cassette protein, multi-drug resistance proteins, multi-drug resistance-associated proteins or organic anion transporting polypeptides.

9. A transgenic mouse, tissue or cells derived therefrom according to claim 8 wherein the human DNA encoding a drug transporter protein encodes MDR1 or MRP2.

10. A transgenic mouse, tissue or cells derived therefrom according to any preceding claim wherein the human DNA sequences include coding sequences operatively linked to human regulatory DNA sequences.

11. A transgenic mouse, tissue or cells derived therefrom according to any one of the preceding claims, incorporating:
(i) a human DNA sequence encoding a PXR and/or a CAR transcription factor,
(ii) a human DNA sequence encoding the CYP3A4 enzyme; and
(iii) a human DNA sequence encoding the MDR1 protein.

12. A transgenic mouse, tissue or cells derived therefrom according to any one of the preceding claims, wherein a promoter of the transcription factor and/or phase 1 and/or phase 2 drug-metabolising enzyme and/or drug transporter protein is linked to a reporter which allows monitoring for the relative regulation of at least one enzyme involved in drug/xenobiotic disposition.

13. A method of introducing at least one functional human transcription factor into a mouse cell whose own endogenous equivalent gene(s) expressing the aforesaid protein have been rendered inactive, the mouse cell derived therefrom further incorporating at least one or more of the following further human DNA sequences selected from the group comprising:
(i) a DNA sequence encoding a phase-1 drug metabolising enzyme;
(ii) a DNA sequence encoding a phase-2 drug-metabolising enzyme; and/or
(iii) a DNA sequence encoding a drug transporter protein
and whose own endogenous equivalent genes expressing the aforesaid proteins have been rendered inactive;
the method comprising introducing DNAs encoding said human transcription factor, phase-1 and 2 drug metabolising enzyme and/or drug transporter protein such that said human expression products remain functional where the mouse cell's own endogenous genes are rendered inactive, and
wherein the mouse genes whose protein products are analogous to the protein products of the introduced human DNA sequences are deleted by either direct targeting with their human counterparts or by flanking these genes or gene clusters with recognition sites and subsequent recombinase-mediated deletion, such that the human DNA sequence is inserted at the point in the genome where the endogenous equivalent gene naturally occurs; and
wherein the cell is humanised for both PXR and CAR alone or in combination.

14. A method according to claim 13, wherein the human genes are at least partially conserved within a construct.

15. A method according to any one of claims 13-14, wherein the PXR construct retains the intron-exon structure between exons 4 and 6.

16. A method according to any one of claims 13-15, wherein the CAR construct retains the intron-exon structure between exons 2 and 9.

17. A method according to any one of claims 13 to 16, wherein the mouse cell is produced *de novo.*

18. A method according to claim 17 wherein DNA sequences are deleted by either direct targeting with their human counterparts or by flanking these genes or gene clusters with recognition sites and subsequent recombinase-mediated deletion.

19. A method according to any one of claims 13 to 18 further comprising the step of introducing a reporter gene DNA sequence so that expression products can be measured by assay of transcription or translation products.

20. Use of a transgenic mouse, tissues and/or cells according to any one of claims 1-12 to investigate xenobiotic metabolism or toxicity in said transgenic mouse, tissues and/or cells derived therefrom or metabolism and/or biosynthesis of endogenous compounds; to investigate function and regulation of human mechanisms of xenobiotic metabolism, disposition and toxicity to be studied in gastrointestinal tract, blood-brain barrier, liver, kidney in a single animal, tissue or cell derived therefrom; to investigate the effects of human metabolism, disposition or toxicity on anti-tumour effects of a drug in a mouse xenograft experiment by expressing humanised metabolic pathways in a mouse grafted tumour cell line and/or in the host mouse animal.

## Patentansprüche

1. Transgene Maus, davon stammendes Gewebe oder davon stammende Zellen, die in ihrem Genom mindestens eine menschliche DNA-Sequenz enthält/enthalten, die mindestens einen Transkriptionsfaktor unter der Kontrolle eines Transkriptionsfaktor-Promotors codiert, und deren endogene äquivalente Gene außer Kraft gesetzt worden sind, wobei die Maus, das davon stammende Gewebe oder die davon stammenden Zellen außerdem mindestens eine oder mehrere der folgenden weiteren menschlichen DNA-Sequenzen enthält/enthalten, ausgewählt aus der Gruppe der folgenden Sequenzen:
(i) eine DNA-Sequenz, die ein Arzneimittel-metabolisierendes Phase-1-Enzym codiert,
(ii) eine DNA-Sequenz, die ein Arzneimittel-metabolisierendes Phase-2-Enzym codiert, und/oder
(iii) eine DNA-Sequenz, die ein Arzneimittel-Transporterprotein codiert,
und deren endogenes äquivalentes Gen oder endogene äquivalente Gene außer Kraft gesetzt worden ist/sind,
wobei die menschliche Sequenz, die mindestens einen Transkriptionsfaktor codiert, an der Stelle im Genom inseriert wird, an der das endogene äquivalente Gen natürlicherweise vorkommt, und
wobei der Transkriptionsfaktor aus der Gruppe ausgewählt wird, die den Pregnan-X-Rezeptor (PXR/SXR) und den konstitutiven Androstan-Rezeptor (CAR) oder eine Kombination davon umfasst.

2. Transgene Maus, davon stammendes Gewebe oder davon stammende Zellen nach Anspruch 1, wobei die menschliche DNA, die einen Transkriptionsfaktor codiert, sowohl den Pregnan-X-Rezeptor (PXR/SXR) als auch den konstitutiven Androstan-Rezeptor (CAR) umfasst.

3. Transgene Maus, davon stammendes Gewebe oder davon stammende Zellen nach einem vorhergehenden Anspruch, wobei die menschliche DNA, die ein Arzneimittel-metabolisierendes Phase-1-Enzym codiert, aus der Gruppe ausgewählt wird, die die P450-Cytochrome umfasst.

4. Transgene Maus, davon stammendes Gewebe oder davon stammende Zellen nach Anspruch 3, wobei das P450 irgendeine oder mehrere der folgenden Isoformen umfasst: CYP3A4, CYP2C9, CYP2C19, CYP2D6, CYP1A2, CYP2C8 und CYP2B6.

5. Transgene Maus, davon stammendes Gewebe oder davon stammende Zellen nach Anspruch 3 oder Anspruch 4, wobei die menschliche DNA, die ein Arzneimittel-metabolisierendes Phase-1-Enzym codiert, das CYP3A-und/oder das CYP2C-Gencluster umfasst.

6. Transgene Maus, davon stammendes Gewebe oder davon stammende Zellen nach einem vorhergehenden Anspruch, wobei die menschliche DNA, die ein Arzneimittel-metabolisierendes Phase-2-Enzym codiert, eine oder mehrere der Transferasen umfasst, die aus der Gruppe mit Glucuronyltransferase, Glutathiontransferase, Sulfonyltransferase und Acetyltransferase ausgewählt werden.

7. Transgene Maus, davon stammendes Gewebe oder davon stammende Zellen nach Anspruch 6, wobei die menschliche DNA, die ein Arzneimittel-metabolisierendes Phase-2-Enzym codiert, das UGT1A-Gencluster umfasst.

8. Transgene Maus, davon stammendes Gewebe oder davon stammende Zellen nach einem vorhergehenden Anspruch, wobei die menschliche DNA, die ein Arzneimittel-Transporterprotein codiert, aus der Gruppe mit einem ATP-Bindungskassette-Protein, Multi-Drug-Resistance-Proteinen, Multi-Drug-Resistance-assoziierten Proteinen oder organische-Anionen-transportierenden Polypeptiden ausgewählt wird.

9. Transgene Maus, davon stammendes Gewebe oder davon stammende Zellen nach Anspruch 8, wobei die menschliche DNA, die ein Arzneimittel-Transporterprotein codiert, MDR1 oder MRP2 codiert.

10. Transgene Maus, davon stammendes Gewebe oder davon stammende Zellen nach einem vorhergehenden Anspruch, wobei die menschlichen DNA-Sequenzen codierende Sequenzen enthalten, die funktionsfähig mit menschlichen regulatorischen Sequenzen verknüpft sind.

11. Transgene Maus, davon stammendes Gewebe oder davon stammende Zellen nach einem der vorhergehenden Ansprüche, die/das folgendes enthält/enthalten:
(i) eine menschliche DNA-Sequenz, die einen PXR-und/oder einen CAR-Transkriptionsfaktor codiert,
(ii) eine menschliche DNA-Sequenz, die das CYP3A4-Enzym codiert und
(iii) eine menschliche DNA-Sequenz, die das MDR1-Protein codiert.

12. Transgene Maus, davon stammendes Gewebe oder davon stammende Zellen nach einem der vorhergehenden Ansprüche, wobei ein Promotor des Transkriptionsfaktors und/oder des Arzneimittel-metabolisierenden Phase-1-und/oder Phase-2-Enzyms und/oder des Arzneimittel-Transporterproteins mit einem Reporter verknüpft ist, wodurch eine Überwachung der relativen Regulation mindestens eines Enzyms, das an der Arzneistoff-/Xenobiotika-Disposition beteiligt ist, ermöglicht wird.

13. Verfahren zum Einbringen mindestens eines funktionellen menschlichen Transkriptionsfaktors in eine Mauszelle, derer eigenes endogenes äquivalentes Gen/eigene endogene äquivalente Gene, das/die das genannte Protein exprimiert/exprimieren, inaktiv gemacht worden ist/sind, wobei die davon stammende Mauszelle außerdem mindestens eine oder mehrere der folgenden weiteren menschlichen DNA-Sequenzen enthält, ausgewählt aus der Gruppe der folgenden Sequenzen:
(i) eine DNA-Sequenz, die ein Arzneimittel-metabolisierendes Phase-1-Enzym codiert,
(ii) eine DNA-Sequenz, die ein Arzneimittel-metabolisierendes Phase-2-Enzym codiert und/oder
(iii) eine DNA-Sequenz, die ein Arzneimittel-Transporterprotein codiert,
und deren eigene endogene äquivalente Gene, die die vorstehen genannten Proteine exprimieren, inaktiv gemacht worden ist/sind,
wobei man bei dem Verfahren DNAs, die den menschlichen Transkriptionsfaktor, das Arzneimittelmetabolisierende Phase-1- und Phase-2-Enzym und/oder das Arzneimittel-Transporterprotein codieren, derart einbringt, dass die menschlichen Expressionsprodukte funktionell bleiben, wobei die eigenen endogenen Gene der Mauszelle inaktiv gemacht worden sind, und
wobei die Mausgene, deren Proteinprodukte zu den Proteinprodukten der eingebrachten menschlichen DNA-Sequenzen analog sind, entweder durch direktes Ansteuern mit ihren menschlichen Entsprechungen oder durch Flankieren dieser Gene oder Gencluster mit Erkennungsstellen und anschließende Rekombinase-vermittelte Deletion deletiert worden sind, so dass die menschliche DNA-Sequenz an der Stelle im Genom inseriert wird, an der das endogene äquivalente Gen natürlicherweise vorkommt, und
wobei die Zelle sowohl für PXR als auch CAR allein oder in Kombination humanisiert ist.

14. Verfahren nach Anspruch 13, wobei die menschlichen Gene zumindest teilweise in einem Konstrukt konserviert werden.

15. Verfahren nach einem der Ansprüche 13-14, wobei das PXR-Konstrukt die Intron-Exon-Struktur zwischen den Exons 4 und 6 beibehält.

16. Verfahren nach einem der Ansprüche 13-15, wobei das CAR-Konstrukt die Intron-Exon-Struktur zwischen den Exons 2 und 9 beibehält.

17. Verfahren nach einem der Ansprüche 13 bis 16, wobei die Mauszelle de novo hergestellt wird.

18. Verfahren nach Anspruch 17, wobei DNA-Sequenzen entweder durch direktes Ansteuern mit ihren menschlichen Entsprechungen oder durch Flankieren dieser Gene oder Gencluster mit Erkennungsstellen und anschließende Rekombinase-vermittelte Deletion deletiert werden.

19. Verfahren nach einem der Ansprüche 13 bis 18, bei dem man außerdem in einem Schritt eine Reportergen-DNA-Sequenz einbringt, so dass Expressionsprodukte durch einen Assay von Transkriptions- oder Translationsprodukten gemessen werden können.

20. Verwendung einer transgenen Maus, von Geweben und/oder Zellen nach einem der Ansprüche 1-12 zur Untersuchung des Xenobiotika-Stoffwechsels oder der Xenobiotika-Toxizität in der transgenen Maus, davon stammenden Geweben und/oder Zellen oder des Stoffwechsels und/oder der Biosynthese endogener Verbindungen; zur Untersuchung der Funktion und Regulation menschlicher Mechanismen des Xenobiotika-Stoffwechsels, der Xenobiotika-Disposition und -Toxizität, die im Gastrointestinaltrakt, an der Blut-Hirn-Schranke, in der Leber, Niere in einem einzelnen Tier, einem davon stammenden Gewebe oder einer davon stammenden Zelle untersucht werden sollen; zur Untersuchung der Wirkungen des Stoffwechsels, der Disposition oder Toxizität beim Menschen auf Antitumorwirkungen eines Arzneistoffs in einem Maus-Xenotransplantat-Experiment, indem man humanisierte Stoffwechselwege in einer in Maus transplantierten Tumorzelllinie und/oder im Maus-Wirtstier exprimiert.

## Revendications

1. Souris transgénique ou bien tissu ou cellules dérivé(es) de celle-ci incorporant dans son génome au moins une séquence d'ADN humain qui code pour au moins un facteur de transcription, étant sous le contrôle d'un promoteur de facteur de transcription, et dont les gènes endogènes équivalents ont été annulés, la souris ou le(s) tissu ou cellules dérivé(es) de celle-ci incorporant en outre au moins une ou plusieurs des séquences d'ADN humain supplémentaires choisies dans le groupe comprenant :
(i) une séquence d'ADN codant pour une enzyme de métabolisation d'une substance médicamenteuse de phase 1 ;
(ii) une séquence d'ADN codant pour une enzyme de métabolisation d'une substance médicamenteuse de phase 2 ; et/ou
(iii) une séquence d'ADN codant pour une protéine de transport d'une substance médicamenteuse
et dont un ou des gène(s) endogène(s) équivalent(s) a (ont) été annulé(s) ;
dans laquelle la séquence humaine codant pour le au moins un facteur de transcription est insérée au niveau du point du génome où existe naturellement le gène endogène équivalent ; et
dans laquelle le facteur de transcription est choisi dans le groupe comprenant le récepteur de pregnane X (PXR/SXR) et le récepteur d'androstane constitutif (CAR) ou une combinaison de ceux-ci.

2. Souris transgénique ou bien tissu ou cellules dérivé(es) de celle-ci selon la revendication 1, dans laquelle l'ADN humain qui code pour un facteur de transcription comprend à la fois le récepteur de pregnane X (PXR) et le récepteur d'androstane constitutif (CAR).

3. Souris transgénique ou bien tissu ou cellules dérivé(es) de celle-ci selon l'une quelconque des revendications précédentes, dans laquelle l'ADN humain qui code pour une enzyme de métabolisation d'une substance médicamenteuse de phase 1 est choisi dans le groupe comprenant les cytochromes P450.

4. Souris transgénique ou bien tissu ou cellules dérivé(es) de celle-ci selon la revendication 3, dans laquelle le P450 comprend une quelconque isoforme ou plus parmi les suivantes : CYP3A4, CYP2C9, CYP2C19, CYP2D6, CYP1A2, CYP2C8 et CYP2B6.

5. Souris transgénique ou bien tissu ou cellules dérivé(es) de celle-ci selon la revendication 3 ou la revendication 4, dans laquelle l'ADN humain qui code pour une enzyme de métabolisation d'une substance médicamenteuse de phase 1 comprend les groupes géniques CYP3A et/ou CYP2C.

6. Souris transgénique ou bien tissu ou cellules dérivé(es) de celle-ci selon l'une quelconque des revendications précédentes, dans laquelle l'ADN humain qui code pour une enzyme de métabolisation d'une substance médicamenteuse de phase 2 comprend une quelconque transférase ou plus choisie(s) dans le groupe comprenant une glucuronyl transférase, une glutathion transférase, une sulfonyl transférase et une acétyl transférase.

7. Souris transgénique ou bien tissu ou cellules dérivé(es) de celle-ci selon la revendication 6, dans laquelle l'ADN humain qui code pour une enzyme de métabolisation d'une substance médicamenteuse de phase 2 comprend le groupe génique UGT1A.

8. Souris transgénique ou bien tissu ou cellules dérivé(es) de celle-ci selon l'une quelconque des revendications précédentes, dans laquelle l'ADN humain qui code pour une enzyme de transport d'une substance médicamenteuse est choisi dans le groupe comprenant une protéine de cassette de liaison à l'ATP, des protéines de résistances croisées, des protéines liées à une résistance croisée ou des polypeptides de transport d'anions organiques.

9. Souris transgénique ou bien tissu ou cellules dérivé(es) de celle-ci selon la revendication 8, dans laquelle l'ADN humain codant pour une enzyme de transport d'une substance médicamenteuse code pour un MDR1 ou un MRP2.

10. Souris transgénique ou bien tissu ou cellules dérivé(es) de celle-ci selon l'une quelconque des revendications précédentes, dans laquelle les séquences d'ADN humain comprennent des séquences codantes étant liées, de manière opérationnelle, à des séquences d'ADN humain de régulation.

11. Souris transgénique ou bien tissu ou cellules dérivé(es) de celle-ci, selon l'une quelconque des revendications précédentes, incorporant ;
(i) une séquence d'ADN humain codant pour un facteur de transcription PXR ou CAR,
(ii) une séquence d'ADN humain codant pour l'enzyme CYP3A4 ; et
(iii) une séquence d'ADN humain codant pour la protéine MDR1.

12. Souris transgénique ou bien tissu ou cellules dérivé(es) de celle-ci selon l'une quelconque des revendications précédentes, dans laquelle un promoteur du facteur de transcription et/ou une enzyme de métabolisation d'une substance médicamenteuse de phase 1 et/ou de phase 2 et/ou une protéine de transport d'une substance médicamenteuse est lié(e) à un rapporteur, ce qui permet le suivi de la régulation relative d'au moins une enzyme impliquée dans une disposition de substance médicamenteuse/xénobiotique.

13. Procédé d'introduction d'au moins un facteur de transcription humain fonctionnel dans une cellule de souris dont son (ses) propre(s) gène(s) endogène(s) équivalent(s), qui expriment la protéine précitée, a (ont) été rendu(s) inactif(s), la cellule de souris dérivée de celle-ci incorporant en outre au moins une ou plusieurs des séquences d'ADN humain supplémentaires choisies dans le groupe comprenant :
(i) une séquence d'ADN codant pour une enzyme de métabolisation d'une substance médicamenteuse de phase 1 ;
(ii) une séquence d'ADN codant pour une enzyme de métabolisation d'une substance médicamenteuse de phase 2 ; et/ou
(iii) une séquence d'ADN codant pour une protéine de transport d'une substance médicamenteuse
et dont ses propres gènes endogènes équivalents, qui expriment les protéines précitées, ont été rendus inactifs ;
le procédé comprenant l'introduction d'ADN codant pour ledit facteur de transcription humain, ladite enzyme de métabolisation d'une substance médicamenteuse de phase 1 et 2 et/ou ladite protéine de transport d'une substance médicamenteuse, de sorte que lesdits produits de l'expression humaine restent fonctionnels là où les propres gènes endogènes de la cellule de souris sont rendus inactifs et
dans lequel les gènes de souris dont les produits protéiques sont analogues aux produits protéiques des séquences d'ADN humain introduites sont supprimés soit par un ciblage direct, avec leurs contreparties humaines, soit en flanquant ces gènes ou ces groupes de gènes de sites de reconnaissance et suivi d'une délétion ayant lieu par médiation d'une recombinase, de sorte que la séquence d'ADN humain soit insérée au niveau du point du génome où existe naturellement le gène endogène équivalent ; et
dans lequel la cellule est humanisée à la fois pour les PXR et CAR individuellement ou en combinaison.

14. Procédé selon la revendication 13, dans lequel les gènes humains sont au moins partiellement conservés au sein d'un construit.

15. Procédé selon l'une quelconque des revendications 13 à 14, dans lequel le construit à PXR conserve la structure d'intron-exon entre les exons 4 et 6.

16. Procédé selon l'une quelconque des revendications 13 à 15, dans lequel le construit à CAR conserve la structure d'intron-exon entre les exons 2 et 9.

17. Procédé selon l'une quelconque des revendications 13 à 16, dans lequel la cellule de souris est produite par voie *de novo.*

18. Procédé selon la revendication 17, dans lequel des séquences d'ADN sont supprimées soit par un ciblage direct, avec leurs contreparties humaines, soit en flanquant ces gènes ou ces groupes de gènes de sites de reconnaissance et suivi d'une délétion ayant lieu par médiation d'une recombinase.

19. Procédé, selon l'une quelconque des revendications 13 à 18, comprenant en outre l'étape d'introduction d'une séquence d'ADN d'un gène rapporteur, de sorte que les produits d'expression puissent être mesurés par un dosage des produits de transcription ou de traduction.

20. Utilisation d'une souris transgénique, des tissus et/ou cellules selon l'une quelconque des revendications 1 à 12, pour étudier le métabolisme ou la toxicité d'une substance xénobiotique dans ladite souris transgénique ou lesdits tissus et/ou lesdites cellules dérivé(e)s de celle-ci ou bien le métabolisme et/ou la biosynthèse de composés endogène ; pour étudier le fonctionnement et la régulation de mécanismes humains du métabolisme, de la disposition et la toxicité de substances xénobiotiques à étudier dans le tractus gastro-intestinal, la barrière hémato-encéphalique, le foie, le rein dans un animal seul, un tissu ou une cellule dérivé(e) de celui-là ; pour étudier les effets du métabolisme, de la disposition ou la toxicité humain(e) sur les effets anti-tumoraux d'une substance médicamenteuse dans une expérience de xénogreffe chez la souris, en exprimant des voies métaboliques humanisées dans une lignée de cellules tumorales greffées de souris et/ou dans l'animal hôte murin.
